# EUROPEAN PATENT SPECIFICATION

(11) **EP 3 013 400 B1**
(45) Date of publication and mention of the grant of the patent: **05.04.2017**
(21) Application number: 14744689.2
(22) Date of filing: 27.06.2014
(51) Int. Cl.: A61M 16/00, F16K 31/06, F04D 25/08

(54) **VENTILATOR SYSTEM**
VENTILATORSYSTEM
SYSTÈME DE VENTILATEUR

(30) Priority: 28.06.2013 US 201313931465; 28.06.2013 US 201313931486; 28.06.2013 US 201313931566; 28.06.2013 US 201313931418; 28.06.2013 US 201313931496
(43) Date of publication of application: 04.05.2016
(73) Proprietor: CareFusion 303, Inc., San Diego, CA 92130 (US)
(72) Inventor: DESILVA, Adrian D., Riverside, CA 92506 (US); VU, Huy Thanh, Westminster, CA 92683 (US); LE, Richard, San Diego, CA 92130 (US); MILLER, Jeffrey Harold, Irvine, CA 92619 (US); GONZALEZ, Hector, Corona, CA 92883 (US); VALDEZ, Raul, Eastvale, CA 92880 (US); WANG, Chiun, Cypress, CA 90630 (US); WILLIAMS, Malcolm R., San Clemente, CA 92674 (US); DUQUETTE, Steven, Laguna Niguel, CA 92677 (US)
(74) Representative: Epping - Hermann - Fischer
(86) International application number: PCT/US2014/044743
(87) International publication number: WO 2014/210566

(56) References cited:
- WO-A1-2006/024532
- US-A- 4 825 904
- US-A- 5 265 594
- US-B1- 6 622 724

## Description

### Cross-references to related applications

This application is a continuation-in-part application of U.S. patent application 13/931,465, entitled "LOW-NOISE BLOWER," filed June 28, 2013, attorney docket number 080625-0422, U.S. patent application 13/931,486, entitled "FLOW SENSOR," filed June 28, 2013, attorney docket number 080625-0423, U.S. patent application 13/931,566, entitled "MODULAR FLOW CASSETTE," filed June 28, 2013, attorney docket number 080625-0424, U.S. patent application 13/931,418, entitled "VENTILATOR EXHALATION FLOW VALVE," filed June 28, 2013, attorney docket number 080625-0425, and U.S. patent application 13/931,496, entitled "FLUID INLET ADAPTER," filed June 28, 2013, attorney docket number 080625-0427.

This application is related to co-pending application U.S. patent application 14/318,285, entitled "FLUID INLET ADAPTER," attorney docket number 080625-0657, and U.S. patent application 14/318,274, entitled "VENTILATOR FLOW VALVE," attorney docket number 080625-0658.

### BACKGROUND

### Field

The present disclosure generally relates to ventilation systems and, in particular, to a modular ventilation system.

### Description of the Related Art

Patients with respiratory injury, such as chronic respiratory failure, may be provided with a ventilator or respirator to assist with their breathing or, in severe cases, take over the breathing function entirely. Ventilators typically provide a flow of air, or other breathing gases, at an elevated pressure during an inhalation interval, followed by an exhalation interval where the pressurized air is diverted so that the air within the patient's lungs can be naturally expelled. The inhalation interval may be initiated upon detection of a patient's natural inhalation or by the ventilator.
Ventilators are available in a variety of sizes with different ranges of air flows and pressures that can be provided. For example, a neonatal patient will require a much lower pressure and volume of air per breath than an adult.
In some respirators that use a blower to pressurize the gas provided to the patient, the blowers that are used are loud and the noise level in the patient's room is commonly 65 dB or more. This level of noise may disrupt the patient's rest and sleep as well as cause fatigue for the caregiver and may further obstruct diagnosis and monitoring of the patient by masking the natural breathing noises that provide an indication of the patient's condition.
Some respirators may be configured to accept one or more breathing gases, for example "pure oxygen" or "heliox 80/20" (a mixture of 80% helium with 20% oxygen) from external sources. The exact gas mixture delivered to the patient, however, may be a mixture of various breathing gases since the specific percentage required for a particular patient may not be commercially available and must be custom mixed in the respirator. It is important to provide precisely the specified flow rate of gas to the patient, particularly for neonatal patients whose lungs are small and very susceptible to damage from overinflation.
US 6,622,724 B1 describes a pressure support system including a source of breathing gas and a pressure generator that receives gas from the gas source and elevates the pressure of the gas to produce a flow of breathing gas at a pressure that is greater than ambient pressure. The pressure generator includes an impeller disposed within a housing. The impeller includes a plurality of backward curved impeller blades attached to an upper face of the impeller body. Each impeller blade extends from a leading end, positioned generally adjacent hub, to a trailing end at a perimeter of the impeller. A flow control valve is provided downstream of the impeller to exhaust a portion of the flow of gas via an exhaust vent, thereby controlling the pressure or flow delivered to the patient.

### SUMMARY

Described herein are ventilators and ventilator components. The ventilator components may be modular such that ventilators may comprise various combinations of ventilator components described herein. The modular ventilator components may be removable or otherwise interchangeable.
In certain embodiments, a ventilator is disclosed comprising a blower and a flow control device, wherein the blower is in fluid communication with the flow control device. The blower comprises a housing defining an impeller cavity, an impeller plate disposed within the impeller cavity and comprising an outside edge and one or more vanes disposed on the impeller plate and comprising a leading surface and a trailing surface connecting at a tip, wherein the leading surface comprises a first portion abutting a second portion, the first portion extends from the tip with a first radius, the second portion extends from the tip with a second radius, the first radius is smaller than the second radius. The flow control device comprises a fixed magnetic field, a drive coil configured to move within the fixed magnetic field in response to a low frequency signal and configured to receive a high frequency signal, a detection coil adjacent the drive coil and configured to detect the high frequency signal in the drive coil, the detected high frequency signal corresponding to a position of the drive coil, a processor coupled to the high frequency source and the low frequency source and configured to receive the detected high frequency signal form the detection coil, a seal configured to move based on the position of the drive coil, and a valve orifice defining a valve seat and a variable opening, the variable opening being adjustable based on a position of the seal relative to the valve seat.

In certain embodiments, a ventilator is disclosed comprising a flow cassette and a fluid inlet adapter. The flow cassette comprises a fluid passage terminating at an inlet and an outlet, a temperature sensor disposed within the fluid passage and configured to detect a temperature, a flow rate sensor disposed within the fluid passage and configured to detect a flow rate, and a processor configured to determine a compensated flow rate based on the temperature and the flow rate. The fluid inlet adapter comprises a first inlet end configured to connect to a fluid source, a second inlet end configured to removably connect to the inlet of the flow cassette, a latching component configured to secure the fluid inlet adapter to the flow cassette, and a machine-readable indicator for identifying the fluid source.

In certain embodiments, a ventilator is disclosed comprising a flow sensor comprising a fluid passage, a flow restriction disposed within the fluid passage such that a fluid passing through the fluid passage must pass through the flow restriction, a first pressure sensor coupled to a first end of the fluid passage and configured to detect a first pressure, a second pressure sensor coupled to a second end of the fluid passage and configured to detect a second pressure, such that the flow restriction is between the first and second pressure sensors, a temperature sensor coupled to the fluid passage and configured to detect a temperature, and a flow sensor processor configured to determine a compensated flow rate based at least on the first pressure, the second pressure, and the temperature.

In certain embodiments, a blower is disclosed that has an impeller comprising an impeller plate and a plurality of blades each attached to the impeller plate. Each blade has a tip and a leading surface that comprises a first portion proximate to the tip. The first portion has a first radius that is within a range of 0.03-0.20 inch.

In certain embodiments, an impeller is disclosed that has an impeller plate having an outside edge with a first radius and a plurality of blades attached to the impeller plate. Each of the plurality of blades comprises a tip at the outside edge and a leading surface with a first portion extending from the tip and a second portion that extends from the first portion with a second radius that is within the range of 0.14-0.16 inch.

Described herein are ventilators having a valve that is a software-controlled valve used to adjust the flow of gas passing through a port of the ventilator. The valve is controlled by a software control signal and works in conjunction with a ventilator's gas delivery subsystems to maintain user set pressure control levels. In continuous positive airway pressure ("CPAP") therapy, the valve preferably helps maintain a set pressure.

Described herein are ventilators having an exhalation valve that is a software-controlled valve used to adjust the flow of gas passing through an expiratory port of the ventilator to the outside environment. The exhalation valve is controlled by a software control signal and works in conjunction with a ventilator's gas delivery subsystems to maintain user set pressure control levels. In CPAP therapy, the exhalation valve preferably maintains a set pressure, and outlet flow is controlled at a specified target bias flow rate. Additional (demand) flow is provided to maintain the pressure in the event of patient inspiratory flow exceeding the bias flow.

Some implementations described herein relate to a flow control device comprising a high frequency source configured to generate a high frequency signal, a low frequency source configured to generate a low frequency signal, and a fixed magnetic field. The flow control device further comprises a drive coil configured to move within the fixed magnetic field in response to the low frequency signal and configured to receive the high frequency signal, and a detection coil adjacent the drive coil and configured to detect the high frequency signal in the drive coil. The detected high frequency signal corresponds to a position of the drive coil. The flow control device further comprises a processor coupled to the high frequency source and the low frequency source and configured to receive the detected high frequency signal from the detection coil. The flow control device further comprises a seal configured to move based on the position of the drive coil, and a valve orifice defining a valve seat and a variable opening. The variable opening is adjustable based on a position of the seal relative to the valve seat.

Described herein are ventilator systems that include, for example, a first valve connected to a supply channel. The first valve comprises a first high frequency source configured to generate a first high frequency signal, a first low frequency source configured to generate a first low frequency signal, and a first fixed magnetic field. The first valve further comprises a first drive coil configured to move within the first fixed magnetic field in response to the first low frequency signal and configured to receive the first high frequency signal, and a first detection coil adjacent the first drive coil and configured to detect the first high frequency signal in the drive coil. The detected first high frequency signal corresponds to a position of the first drive coil. The first valve further comprises a first processor coupled to the first high frequency source and the first low frequency source and configured to receive the detected first high frequency signal from the first detection coil. The first valve further comprises a first seal configured to move based on the position of the first drive coil, and a variable first valve orifice defining a first valve seat. The first valve orifice is adjustable based on a position of the first seal relative to the first valve seat.

Described herein are also methods for adjusting pressure in a ventilator line. Some methods include sending a high frequency signal and a low frequency signal to a drive coil. The low frequency signal causes the drive coil to move within a fixed magnetic field, and the drive coil causes a seal to adjust a variable valve orifice of the valve. The methods also include detecting the high frequency signal in the drive coil, determining a velocity of the drive coil based on the detected high frequency signal, and modifying the low frequency signal based on the determined velocity of the drive coil.

Some embodiments described herein relate to a valve that includes a valve orifice with an adjustable opening; a fixed magnetic field; a force coil configured to be moved within the fixed magnetic field in response to a low frequency current; a current amplifier configured to direct a summed low frequency current and a high frequency current into the force coil; a feedback coil configured to detect the high frequency current in the force coil, the detected high frequency current having a magnitude that is proportional to a force coil position within the fixed magnetic field. The valve can also include a processor configured (i) to receive data relating to the position of the force coil and (ii) to send instructions to the current amplifier; and a diaphragm configured to adjust the valve orifice opening based on the position of the force coil.

Described herein are ventilator systems that include, for example, a gas source configured to provide a gas to a patient via a supply channel; an exhaust channel configured to direct exhaust gas from the patient; and an exhaust valve. The exhaust valve may include a force coil configured to be moved within a fixed magnetic field in response to a low frequency current; a current amplifier configured to direct a summed low frequency current and a high frequency current into the force coil; a feedback coil configured to detect the high frequency current in the force coil; a processor configured (i) to receive data relating to the position of the force coil, (ii) to receive data relating to pressure within the exhaust channel, and (iii) to send instructions to the current amplifier based on the position of the coil and the pressure; and a diaphragm configured to adjust opening of a valve orifice based on the instructions from the processor.

Described herein are also methods for adjusting pressure in a ventilator line. Some methods include the following steps: directing a summed low frequency current and a high frequency current from a current amplifier into a force coil that is configured (i) to be moved within a fixed magnetic field in response to the low frequency current and (ii) to control a diaphragm to adjust opening of a valve orifice; detecting the high frequency current in the force coil, the detected high frequency current having a magnitude that is proportional to a position of the force coil within the fixed magnetic field; detecting the pressure in the ventilator line; and changing the low frequency current to move the force coil within the fixed magnetic field, thereby adjusting the opening of a valve orifice, in response to the detected pressure.

The disclosed fluid inlet adapter provides a fluid inlet that can be configured to accept only one of two possible fluids at a time and provide a machine-readable indication as to which fluid is currently being accepted.

In certain embodiments, an adapter for providing fluid from a fluid source to a device is disclosed. The adapter comprises a housing and an inlet extending through the housing for connecting the fluid source to the device. The inlet comprises a first end for connecting to the fluid source and a second end for connecting to the device. The adapter further comprises a latching component configured to secure the adapter to the device, and a machine-readable indicator for identifying the fluid source. The machine-readable indicator extends away from the first end of the inlet and beyond the second end of the inlet.

In certain embodiments, a flow sensor is disclosed that comprises a flow restriction disposed within a passage such that a fluid passing through the passage must pass through the flow restriction, an upstream pressure sensor coupled to the passage at a point upstream of the flow restriction and configured to measure and provide an upstream pressure of the fluid within the passage, a downstream pressure sensor coupled to the passage at a point downstream of the flow restriction and configured to measure and provide a downstream pressure of the fluid within the passage, a temperature sensor coupled to the passage and configured to measure and provide a temperature of the fluid within the passage, and a flow sensor processor coupled to the upstream and downstream pressure sensors and the temperature sensor and configured to accept measurements therefrom and calculate a compensated flow rate based at least in part on the measured pressures and temperature.

It is advantageous to provide a modular flow cassette that provides accurate flow measurements of a variety of gases and gas mixtures over a range of temperatures and flow rates.

In certain embodiments, a flow cassette is disclosed that has a housing with an inlet and an outlet and a passage therebetween. The flow cassette also has a temperature sensor disposed within the passage and configured to measure the temperature of a fluid flowing through the passage, a flow rate sensor disposed within the passage and configured to measure a flow rate of the fluid flowing through the passage, and a processor coupled to the temperature sensor and flow rate sensor. The processor is configured to accept measurements of temperature and flow rate from the temperature sensor and flow rate sensor, respectively, and provide a compensated flow rate.

For purposes of summarizing the disclosure, certain aspects, advantages, and novel features of the disclosure have been described. It is to be understood that not necessarily all such advantages may be achieved in accordance with any particular embodiment of the disclosure. Thus, the disclosure may be embodied or carried out in a manner that achieves or optimizes one advantage or group of advantages as taught herein without necessarily achieving other advantages taught or suggested.

### BRIEF DESCRIPTION OF THE DRAWINGS

The accompanying drawings, which are included to provide further understanding and are incorporated in and constitute a part of this specification, illustrate disclosed embodiments and together with the description serve to explain the principles of the disclosed embodiments. In the drawings:
FIGS. 1-2 are top and bottom perspective views of an exemplary blower according to certain aspects of the present disclosure.
FIG. 3 is an exploded view of the blower of FIG. 1 according to certain aspects of the present disclosure.
FIG. 4 is a perspective view of an exemplary impeller according to certain aspects of the present disclosure.
FIG. 5 is a perspective view of an exemplary impeller according to certain aspects of the present disclosure.
FIG. 6 is a close-up plan view of a vane tip of the impeller according to certain aspects of the present disclosure.
FIG. 7 is a cross-section of a blower according to certain aspects of the present disclosure.
FIG. 8 is an enlarged view of a portion of FIG. 7 according to certain aspects of the present disclosure.
FIGS. 9A-9C are perspective views of the overmolded top housing according to certain aspects of the present disclosure.
FIG. 10 depicts a patient using an exemplary ventilation system according to certain aspects of the present disclosure.
FIGS. 11A and 11B are front and rear views of an exemplary ventilator according to certain aspects of the present disclosure.
FIG. 12 is a schematic representation of a ventilator according to certain aspects of the present disclosure.
FIGS. 13A-13B are schematic depictions of feedback systems according to certain aspects of the present disclosure.
FIG. 14 illustrates an exemplary schematic arrangement of a control system according to certain aspects of the present disclosure.
FIG. 15A is a cross sectional view of a flow valve according to certain aspects of the present disclosure.
FIG. 15B is a cross sectional view of a flow valve according to certain aspects of the present disclosure.
FIG. 16 is a schematic representation of a ventilator according to certain aspects of the present disclosure.
FIG. 17 shows a flowchart of a process for controlling a flow valve according to certain aspects of the present disclosure.
FIG. 18 illustrates high frequency signals according to certain aspects of the present disclosure.
FIGS. 19-20 are front and back perspective views of exemplary fluid inlet adapters according to certain aspects of the present disclosure.
FIG. 21A is a cross-sectional side view of an exemplary fluid inlet adapter and a device according to certain aspects of the present disclosure.
FIG. 21B is a cross-sectional side view of the exemplary fluid inlet adapter mated with the docking location of the housing according to certain aspects of the present disclosure.
FIGS. 22A-22B depict the position of the handle in exemplary unlatched and latched positions according to certain aspects of the present disclosure.
FIGS. 23 and 24 depict an exemplary inlet adapter configured to accept fluid from two different sources according to certain aspects of the present disclosure.
FIGS. 25-28 depict example connector configurations according to certain aspects of the present disclosure.
FIG. 29 depicts an adapter with one inlet according to certain aspects of the present disclosure.
FIG. 30 depicts adapters with different machine-readable indicators and a flow control device according to certain aspects of the present disclosure.
FIG. 31 depicts an adapter coupled to a flow control device according to certain aspects of the present disclosure.
FIG. 32 depicts frontal views of an adapter and a connector according to certain aspects of the present disclosure.
FIG. 33 depicts front views of an adapter and a flow control device according to certain aspects of the present disclosure.
FIG. 34 is a block diagram of an exemplary flow sensor according to certain aspects of the present disclosure.
FIG. 35A depicts an exemplary flow cassette according to certain aspects of the present disclosure.
FIG. 35B is a cross-section of the flow cassette of FIG. 35A according to certain aspects of the present disclosure.
FIG. 35C is an enlarged view of a portion of FIG. 35B showing an exemplary flow sensor according to certain aspects of the present disclosure.
FIG. 36 is a flow chart of an exemplary flow measurement process according to certain aspects of the present disclosure.
FIG. 37 is a block diagram of an exemplary flow cassette according to certain aspects of the present disclosure.
FIGS. 38A-38B depict an exemplary flow cassette according to certain aspects of the present disclosure.
FIG. 39A is a cross-section of the flow cassette of FIGS. 38A-38B according to certain aspects of the present disclosure.
FIG. 39B is an enlarged view of a portion of FIG. 39A according to certain aspects of the present disclosure.
FIG. 40 is a flow chart of an exemplary configuration process according to certain aspects of the present disclosure.
FIG. 41 is an illustration of a ventilator according to certain aspects of the present disclosure.

### DETAILED DESCRIPTION

In the following detailed description, numerous specific details are set forth to provide a full understanding of the present disclosure. It will be apparent, however, to one ordinarily skilled in the art that embodiments of the present disclosure may be practiced without some of the specific details. In other instances, well-known structures and techniques have not been shown in detail so as not to obscure the disclosure. In the referenced drawings, like numbered elements are the same or essentially similar. Reference numbers may have letter suffixes appended to indicate separate instances of a common element while being referred to generically by the same number without a suffix letter.

While the discussion herein is directed to a ventilator for use in a hospital, the disclosed concepts and methods may be applied to environments, such as a home or long-term care facility, and other fields, such as deep-sea diving, that would benefit from accurate flow measurement of a variety of gas mixtures. Those of skill in the art will recognize that these same features and aspects may also be applied to the sensing and control of other fluids besides medical gases.

Within this document, the term "gas" shall be interpreted to mean both a single material in gaseous form, for example oxygen, and a mixture of two or more gases, for example air or heliox (a mixture of oxygen and helium). A gas may include water or other liquids in the form of vapor or suspended droplets. A gas may also include solid particulates suspended in the gas.

Within this document, the term "pure," when used with reference to a gas, means that the gas meets commonly accepted medical standards for purity and content.

Within this document, the term "temperature sensor" means a device configured to measure temperature and to provide a signal that is related to the measured temperature. A temperature sensor may include electronics to provide a drive current or voltage and/or measure a current or voltage. The electronics may further include conditioning and conversion circuitry and/or a processor to convert the measured value to a signal that may be in analog or digital form.

Within this document, the term "pressure sensor" means a device configured to measure a gas pressure and provide a signal that is related to the measured pressure. A pressure sensor may include electronics to provide a drive current or voltage and/or measure a current or voltage. The electronics may further include conditioning and conversion circuitry and/or a processor to convert the measured value to a signal that may be in analog or digital form. The pressure may be provided in absolute terms or "gauge" pressure, i.e., relative to ambient atmospheric pressure.

While the discussion herein is directed to the provision of compressed air as part of a medical respirator, the disclosed concepts and methods may be applied to other fields that would also benefit from a quiet, portable source of compressed air. For example, conventional leaf blowers that are commonly used to blow leaves and small garden debris into piles are quite loud and a blower of this type may be advantageous in place of the current blowers.

Described herein are ventilators having one or more valves that are software-controlled valves. These valves may be used to adjust the flow of gas passing through a port of the ventilator and can be configured to be positioned on the exhalation side of a ventilation system (meaning in connection with system components that receive exhaled air from a patient) or on an inhalation side of a ventilation system (meaning in connection with system components that provide air to a patient). The valves can be controlled by a software control signal and work in conjunction with a ventilator's gas delivery subsystems to maintain user set pressure control levels. In CPAP therapy, an exhalation valve preferably maintains a set pressure, and outlet flow is controlled at a specified target bias flow rate. Additional (demand) flow may be provided through an inhalation valve to control the pressure.

An exhalation subsystem of a ventilator comprises an exhalation valve, an exhalation flow sensor, and a heated filter and water trap. As explained herein, the exhalation valve is a software-controlled valve that is used to adjust the flow of gas passing through the expiratory port of the ventilator to the outside environment. The exhalation valve is controlled by a software control signal and works in conjunction with a ventilator's gas delivery subsystems to maintain user set pressure control levels.

As explained herein, the exhalation valve operates on the principle of a force balance across a control diaphragm, which may be a disposable valve membrane. In some embodiments, a linear magneto-mechanical actuator controls a force on the diaphragm, which in turn controls the circuit or ventilator line pressure. The force generated by the actuator is based on a command from the software closed-loop controller.

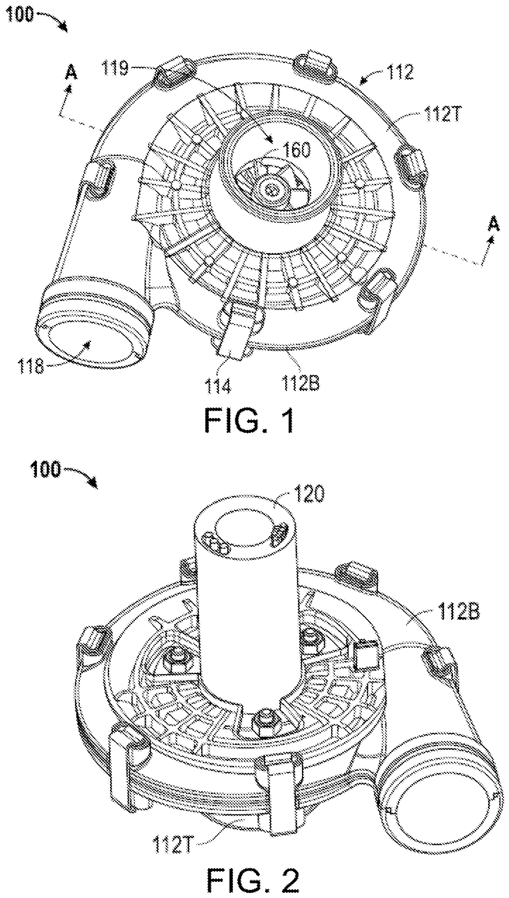

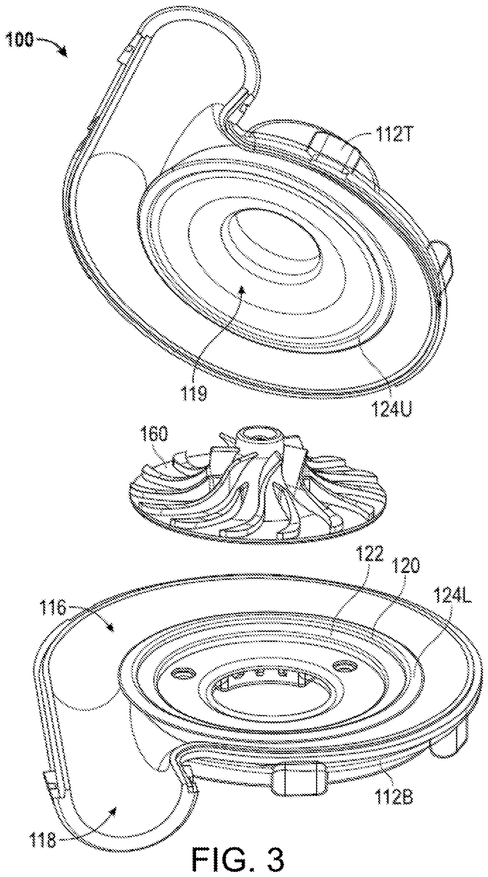

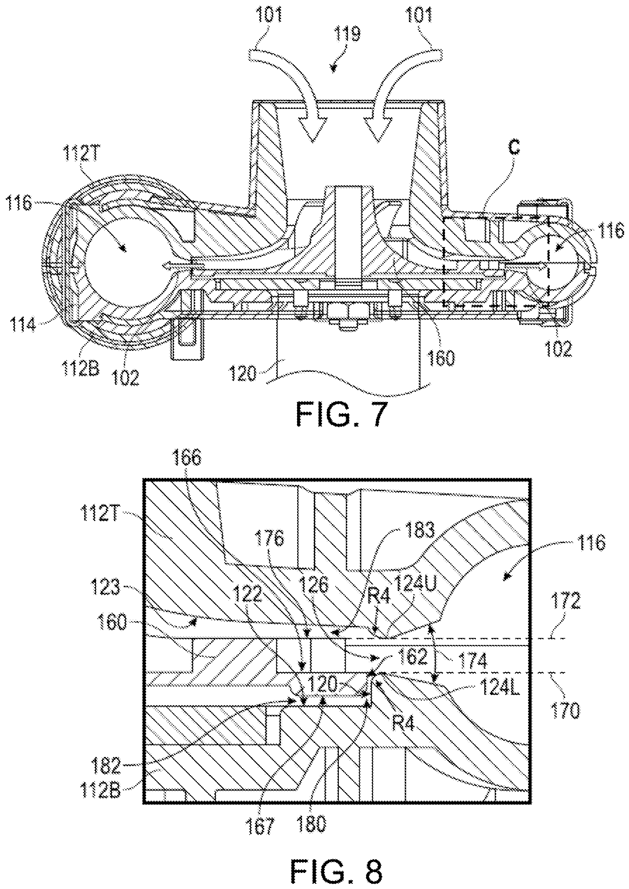

FIGS. 1-2 are top and bottom perspective views of an exemplary blower 100 according to certain aspects of the present disclosure. In the configuration of FIG. 1, the blower 100 draws in ambient air, or other gases if connected to a source of gas, through inlet 119 of housing 112. Impeller 160 may rotate at a variable speed up to 30,000 rotations per minute (rpm), for example, to centrifugally accelerate the air and provide a flow of pressurized air at outlet 118. In this embodiment, the housing 112 comprises two parts, 112T and 112B (see FIG. 3) held together with multiple clips 114. The section line A-A indicates the cross-sectional view of FIG. 7.

FIG. 2 is a perspective view of the blower 100 of FIG. 1 with the blower 100 rotated so as to make the bottom housing portion 112B visible. A motor 120 is attached to the housing 112 and the shaft of the motor (not visible in FIG. 2) passes through the housing 112 and connects to the impeller 160.

FIG. 3 is an exploded view of the blower 100 of FIG. 1 according to certain aspects of the present disclosure. The top housing portion 112T has an impeller cavity 119. The impeller 160 is at least partially disposed within the impeller cavity 119 when the blower 100 is assembled. The housing 112 comprises a collector 116 formed when the top and bottom housing portions 112T, 112B are mated. In this example, the collector 116 is shaped as a volute having a circular cross-sectional profile along a radial plane of the housing 112, wherein the area of the profile monotonically increases as the distance around the volute from the outlet 118 decreases. In certain embodiments, the volute may have a non-circular profile. In certain embodiments, the area of the profile may be constant over a portion of the volute.

The top and bottom housing portions 112T, 112B also respectively include edges 124U, 124L that are proximate to each other when the blower 100 is assembled and surround the impeller cavity so as to cooperatively define a slot (not visible in FIG. 3) that connects the impeller cavity 119 to the collector 116. The lower housing portion 112B also includes a wall 120 and a shelf 122 adjacent to the edge 124L. This region of the blower 100 is described in greater detail with respect to FIG. 7.

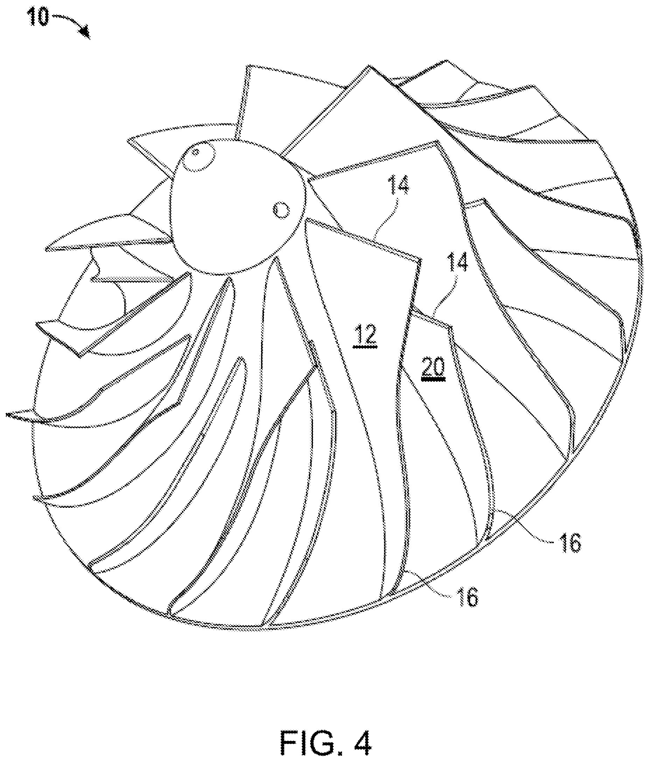

FIG. 4 is a perspective view of a conventional impeller 10. This impeller 10 has a plurality of vanes 12, 20 each having a leading edge 14 and a trailing edge 16, although vanes 20 are shorter than vanes 12 and are commonly referred to as "splitters." Conventional vanes 12, 20 have a three-dimensional curvature with a generally uniform thickness from leading edge 14 to trailing edge 16, with some rounding of the outside corners and filleting of the inside corners.

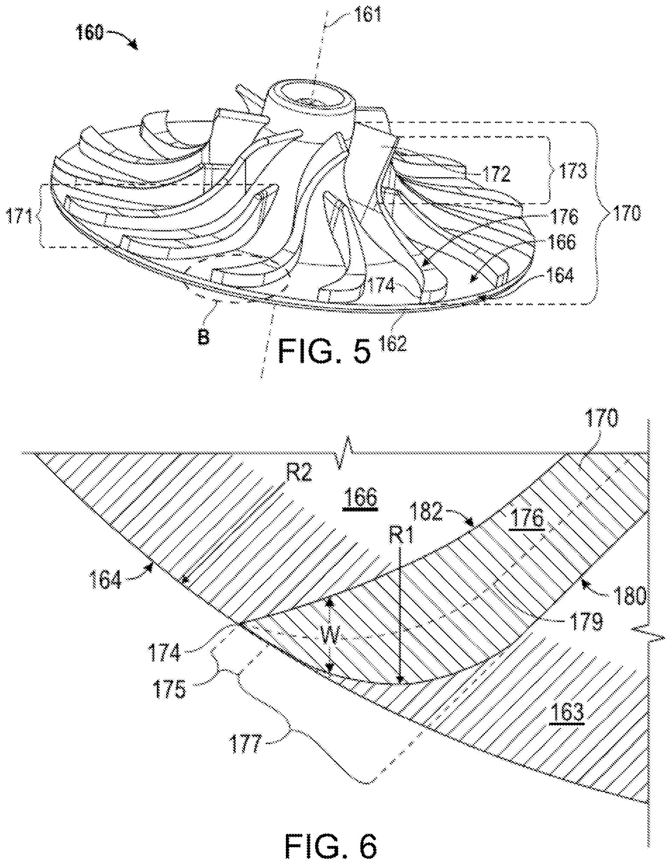

FIG. 5 is a perspective view of an exemplary impeller 160 according to certain aspects of the present disclosure. The impeller 160 comprises an impeller plate 162 having a shaped surface 166 and a circular outside edge 164 that are centered about an axis of rotation 161. In this embodiment, there is a set of long vanes 170 that alternate with a set of splitter vanes 171. The portion of the long vanes 170 that is not present in the splitter vanes 171 is referred to as the "inducer" 173. Each vane 170, 171 has an inlet edge 172, a tip 174 that is proximate to the outside edge 164, and a top surface 176 that is, in this example, flat in a generally circumferential direction about axis 161 while being curved in a generally radial direction. The region indicated by the dashed-line oval labeled "B" is shown in FIG. 6.

FIG. 6 is a close-up plan view of a vane tip 174 of the impeller 160 of FIG. 5 according to certain aspects of the present disclosure. The example vane 170 has a leading surface 180 and a trailing surface 182 that meet at the tip 174. The leading surface 180 comprises a first portion 177 and a second portion 175. In this example, the second portion 175 extends from the tip 174 with a radius R2 that is the same radius as the outside edge 164. The first portion 177 abuts the second portion 175 and continues with a radius R1 that is smaller than R2 and larger than the radii conventionally used to round outside edges. In certain embodiments, the radius R1 may be within the range of 0.03-0.20 inch. In certain embodiments, the radius R1 may be within the range of 0.12-0.18 inch. In certain embodiments, the radius R1 may be within the range of 0.14-0.16 inch. In certain embodiments, the radius R1 may be approximately 0.15 inch.

Without being bound by theory, it is believed that the effect of the radius R1 may be to control the turbulence of the air at the tip 174 and reduce the velocity gradient in the air flow as the air leaves the impeller 160, compared to a conventional vane that abruptly ends at the outside edge with a sizable angle between the leading surface and the outside edge, as is visible in the impeller 10 of FIG. 4. By smoothing this transition over a larger area, e.g. the length of the first portion 177 and possible the second portion 175, and redirecting the air flow direction, the velocity gradient is reduced and the air flow may be less turbulent as the air leaves the impeller 160 and passes through the slot 126.

Each vane 170, 171 has a centerline 179 that is coincident with and bisects the top surface 176 between the leading and trailing surfaces 180, 182. The vanes 170, 171 have a common width W taken perpendicular to the centerline 179, wherein W varies along the centerline 179. At a common distance from the tip 174 along the centerline 179, the width W of each vane 170, 171 will be the same, for at least the length of the splitter vanes 171.

The shape and width of the vanes 170, 171 proximate to the second portion 177 may be selected to simply enable the radius to be as large as R1, compared to the constant-thickness vanes of a conventional impeller. In certain embodiments, the trailing surface 182 has a minimum radius that is greater than the radius of the first portion of the leading surface 180. In certain embodiments, the shape and location of the trailing surface 182 may be chosen to cooperate with the leading surface of the adjacent vane 170, 171 to control the pressure and/or velocity of the air flowing between the leading and trailing surfaces 180,182.

The height of the vanes 170, 171, *i.e.* the distance from the shaped surface 166 to the top surface 176, varies from the inlet edge 172 and tip 174. In certain embodiments, the height is constant from the tip 174 over the first and second portions 177, 175 of the leading surface 180. In certain embodiments, the shaped surface 166 has an outside portion 163 that is proximate to the first and second portions 177, 175 of the leading surface 180. In certain embodiments, the shaped surface 166 is flat and perpendicular to the axis 16 in the outside portion 163.

FIG. 7 is a cross-section taken along section line A-A of the blower 100 of FIG. 1 according to certain aspects of the present disclosure. The arrows 101 and 102 respectively indicate how air is drawn in through the inlet 119 and directed by the impeller 160 into the collector 116. The motor 120 is shown in schematic form and, in this embodiment, the rotor (not shown in FIG. 7) of the motor 120 is directly connected to the impeller 160. The area indicated by the dashed-line box "C" is discussed in greater detail with respect to FIG. 8.

FIG. 8 is an enlarged view of the area C of FIG. 7 according to certain aspects of the present disclosure. The cross-section of impeller 160 shows the top surface 166, a bottom surface 167, and the outside edge 162. The upper edge 124U of the top housing portion 112T and the lower edge 124L of the bottom housing portion 112B are proximate to each other and separated by a slot 126. In this embodiment, the edges 124U and 124L both have a radius R4 on an inside corner nearest to the impeller 160, with angled surfaces that extend outward at an included angle 174 toward the collector 116. The radius R4 improves the efficiency of the nozzle formed by slot 126. The radius R4 of edges 124U and 124L induces the "Coandǎ effect," wherein a flowing gas will tend to follow a curved surface more readily than a sharp edge, in the gas flowing through the slot 126. This redirection of the gas flowing through the slot 126 produces a smoother transition of flow and pressure with lower loss and reduced audible noise. In certain embodiments, R4 may be within the range of 0.01-0.30 inch. In certain embodiments, R4 may be approximately 0.020 inch.

In this example, the reference line 170 is aligned with the peak of the radius R4 of lower edge 124L and the reference line 172 is aligned with the peak of the radius R4 of upper edge 124U. Thus, the slot 126 is defined by the reference lines 170, 172. In this example, the shaped surface 166 at the outside edge 162 is aligned with reference line 170, *i.e.* the lower edge 124L of the slot 126, and the top surfaces 176 of the plurality of blades 170, 171 are each aligned with the reference line 172, *i.e.* the upper edge 124U of the slot 126.

The gap between the top surface 176 and the upper inner surface 123 of the upper housing 112T is a path of potential backflow from the edge 162 of the impeller 160 toward the center. Minimizing the gap 183 between top surface 176 and the upper inner surface 123 reduces this backflow and thereby improves the pressure recovery of the blower 100. In certain embodiments, the gap 183 may be in the range of 0.002-0.150 inch when the impeller 160 is stationary relative to the housing 112. In certain embodiments, the gap 183 may be in the range of 0.005-0.050 inch. In certain embodiments, the gap 180 may be approximately 0.010 inch.

The lower edge 124L has an adjacent wall 120 with a gap 180 between the outside edge 162 and the wall 120. In certain embodiments, the gap 180 may be in the range of 0.0035-0.110 inch when the impeller 160 is stationary relative to the housing 112. In certain embodiments, the gap 180 may be in the range of 0.005-0.050 inch. In certain embodiments, the gap 180 may be approximately 0.0073 inch. In certain embodiments, the radius R2 of the outside edge 162 of the impeller 160 may increase as the rotational velocity of the impeller 160 increases and, therefore, the gap 180 may be reduced when the impeller 160 is rotating relative to the housing 112. In certain embodiments, the impeller 160 may rotate at a rotational velocity of up to 60,000 rotations per minute (rpm) relative to the housing 112 and the gap may be reduced to as little as 0.003 inch. As there will be a boundary layer (not visible in FIG. 8) attached to each of the wall 120 and the outside edge 162, reducing this gap may decrease the portion of the gap wherein airflow may go turbulent, e.g. the portion between the two boundary layers, thereby reducing the acoustic energy generated by the turbulent air.

The wall 120 connects to a shelf 122. There is a gap 182 between the bottom surface 167 of the impeller 160 and the shelf 122 of the bottom housing portion 112B. In certain embodiments, the gap 182 may be less than or equal to 0.020 inch when the impeller 160 is stationary or moving relative to the housing 112B. In certain embodiments, the gap 182 may be less than or equal to 0.050 inch. In certain embodiments, the gap 182 may be less than or equal to 0.020 inch.

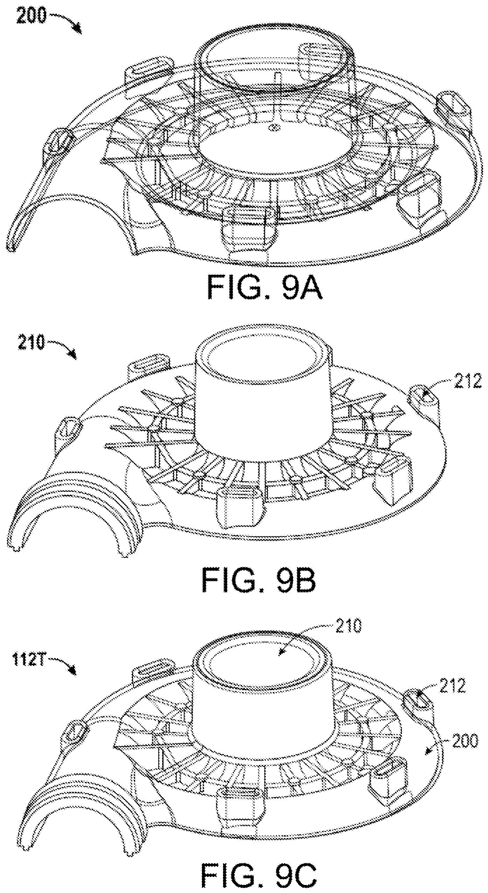

FIGS. 9A-9C are perspective views of the overmolded top housing 112T according to certain aspects of the present disclosure. FIG. 9A depicts a translucent view of a layer 200 of a sound-damping material formed so as to match the external profile of a housing shell 210 shown in FIG. 9B. In this example, the sound-damping layer 200 covers most of the external surface of the housing shell 210 with the exception of the attachment points 212 for the clips 114 (not shown in FIGS. 9A-9C). FIG. 9C shows combined top housing portion 112T with the sound-damping layer 200 on the external surface of the housing shell 210.

In certain embodiments, the sound-damping layer 200 comprises an elastomer, e.g. a silicone or rubber, having poor acoustic transmissibility. In certain embodiments, the sound-damping layer 200 may be overmolded on the housing shell 210. In certain embodiments, the sound-damping layer 200 may be applied to the housing shell 210 by one or more of the processes of transfer molding, spraying, dipping, brushing, curtain coating, or other manual or automated coating application process. In certain embodiments, the sound-damping layer 200 may comprise high-density particles, e.g. steel, that may further reduce the transmissibility of the sound-damping layer 200.

It can be seen that the disclosed embodiments of the blower may provide advantages in size, cost, performance, and reduced noise during operation. The shaping of the leading and trailing surfaces of the vanes near the outside edge of the impeller may reduce the turbulence and velocity gradient in the air flow around the tip of the vanes, thereby reducing the acoustic noise generated by the air flow. The small clearances between portions of the impeller and portions of the housing may further reduce the acoustic noise by decreasing the gaps compared to the depth of the boundary layers, thereby reducing the portion of the gap susceptible to noisy, turbulent flow.

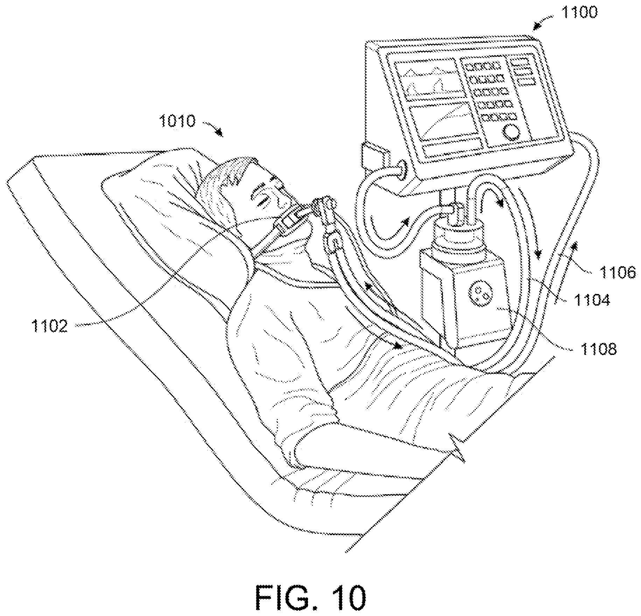

FIG. 10 depicts a patient 1010 using an exemplary ventilation system with a ventilator 1100 according to certain aspects of the present disclosure. The ventilator 1100 may include a blower, such as the blower 100 described herein. The ventilator 1100 operates as a gas source for providing gas to a patient (e.g., for respiration). In this example, the ventilator system includes a supply channel, tube, or "limb" 1104, a return or exhaust channel, tube, or limb 1106, a conditioning module 1108 that may, for example, warm or humidify the air passing through the supply limb 1104. The supply and exhaust limbs 1104, 1106 are both coupled to a patient interface device 1102 that, in this example, is a mask that fits over the mouth of the patient 1010. In other embodiments (not shown in FIG. 10), the patient interface device 1102 may include a nasal mask, an intubation device, or any other breathing interface device as known to those of skill in the art.

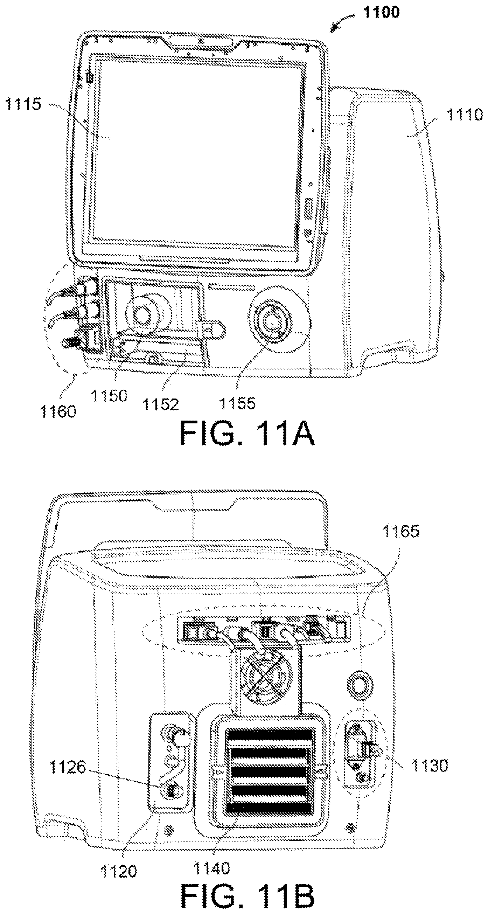

FIGS. 11A and 11B are front and rear views of the ventilator 1100 according to certain aspects of the present disclosure. The ventilator 1100 has a housing 1110 with an attached user interface 1115 that, in certain embodiments, comprises a display and a touchscreen. In FIG. 11A, it can be seen that the front of the housing 1110 includes a supply port 1155 for a supply limb, such as supply limb 1104 in FIG. 10, and a return port 1150 for an exhaust, such as exhaust limb 1106 in FIG. 10. The return port 1150 may be mounted over an access door 1152 that provides access to a filter (not visible in FIG. 11 A) that filters and absorbs moisture from the exhaled breath of the patient 1010. In certain embodiments, there may also be a front connection panel 1160 for connection to external instruments or a network interface cable.

FIG. 11B shows a rear view of the ventilator 1100 with a gas inlet adapter 1120, an air intake port 1140, and a power interface 1130 that may include a power plug connector and a circuit breaker reset switch. There may also be a rear interface panel 1165 for connection to external instruments or a network interface cable. A flow control device, such as a flow cassette described herein or a flow control valve described herein, may be installed within the housing 1110 behind the gas inlet adapter 1120 and in fluid communication between an inlet connector 1126 shown in FIG. 11B and the supply port 1155 shown in FIG. 11A.

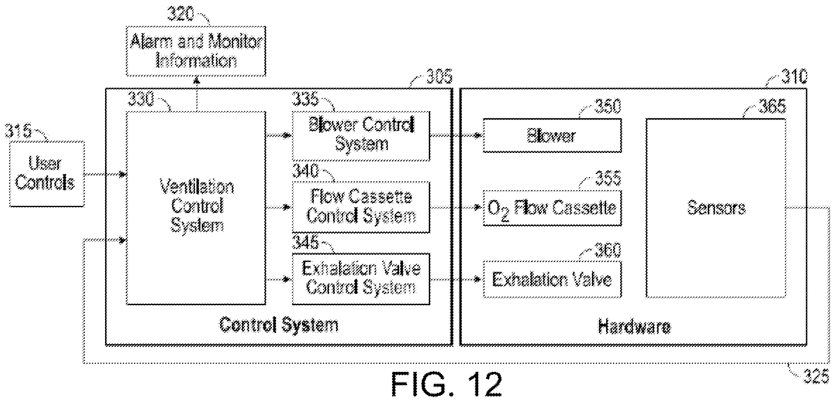

FIG. 12 illustrates a schematic depiction of the ventilator 1100 having a control system 305, system hardware 310, user input 315, output 320, and feedback 325. The control system 305 includes a ventilation control system 330 that receives user input 315. The control system 305 includes hardware control systems that control respective hardware components of the ventilator 1100. For example, the hardware control systems may include a blower control system 335, a flow cassette control system 340, and an exhalation valve control system 345. The blower control system 335 controls a respective blower 350, the flow cassette control system 340 controls a respective flow cassette 355, and the exhalation valve control system 345 controls a respective exhalation valve 360. The blower 350 may correspond to the blower 100 described herein, and may be in fluid communication with the flow cassette 355 and/or the exhalation valve 360. For example, the blower 350 may be before or after the flow cassette 355 or the exhalation valve 360 in a fluid path of the ventilator 1100.

The system hardware 310 includes sensors 365 that detect information from the system hardware 310, for example, the blower 350, the flow cassette 355, and the exhalation valve 360. The sensors 365 produce one or more feedback signals 325 that are received by the ventilation control system 330. The ventilation control system 330 receives the feedback control signals 325 and the user input 315 and sends information to an output 320. The output 320 can include, for example, monitoring information and alarms. The feedback control signals 325 may also be used to provide inputs to the blower control system 335, the flow cassette control system 340, and the exhalation valve control system 345.

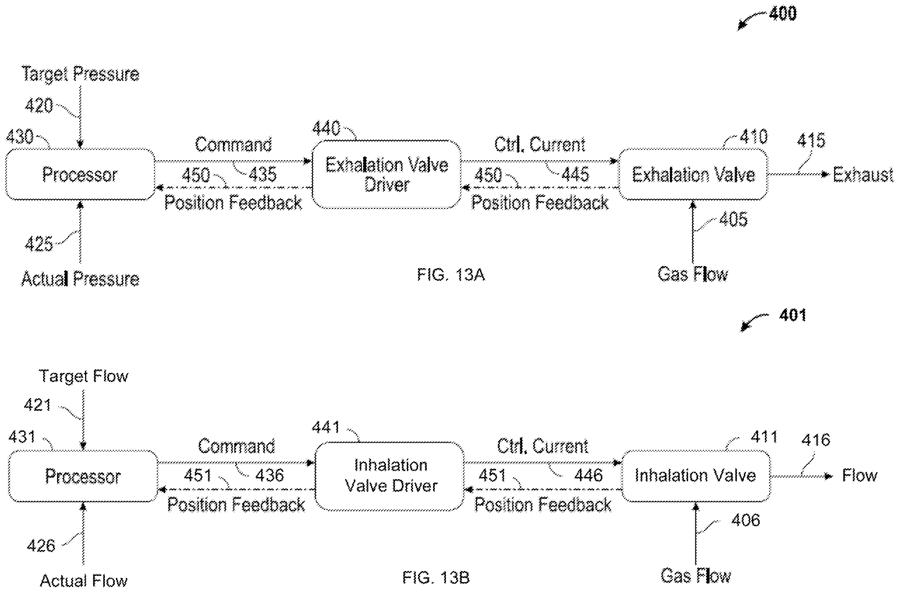

One example of feedback and control of the ventilator 1100 is depicted in FIG. 13A, which illustrates a schematic depiction of an exhalation control feedback system 400 that determines an amount of gas flow 405 that is permitted to pass through an exhalation valve 410. The illustrated embodiment of the feedback system 400 is based on a target pressure 420 and an actual circuit pressure 425 (or a pressure within a line of the ventilator 1100).

As illustrated in FIG. 13A, a processor 430 receives an input signal relating to the actual circuit pressure 425 and compares the actual circuit pressure 425 to the target pressure 420. Based on this comparison, the processor 430 sends a command signal 435 to an exhalation valve driver 440. The exhalation valve driver 440 is configured to control a position of the exhalation valve 410 to regulate the gas flow 405 through the exhalation valve 410. In the illustrated embodiment, the exhalation valve driver 440 sends a control current 445 to the exhalation valve 410 to maintain or adjust the exhalation valve 410 to modify or adjust the pressure within the ventilator line.

For example, if the actual circuit pressure 425 was found to be too high, the processor 430 sends a command 435 to the exhalation valve driver 440 to open the exhalation valve 410 to reduce pressure within the ventilator line. The exhalation valve driver 440, upon receiving the command 435 to relieve pressure, adjusts the control current 445 to the exhalation valve 410 to increase the opening of the exhalation valve 410 and relieve pressure within the ventilator line. As the control current 445 increases the opening of the exhalation valve 410, the processor 430 receives position feedback 450 of the exhalation valve 410 via the exhalation valve driver 440, such that the processor 430 is able to determine the degree to which the exhalation valve 410 is open.

If the actual circuit pressure 425 input to the processor 430 was found to be too low, the processor 430 directs the driver 440 to adjust the control current 445 to the exhalation valve 410 to decrease the opening of the exhalation valve 410 such that pressure within the ventilator line is increased. If the actual circuit pressure 425 input to the processor 430 was found to be at an acceptable level or within an acceptable range, the processor 430 directs the driver 440 to maintain the control current 445 to the exhalation valve 410 to maintain the position of the exhalation valve 410.

Another example of feedback and control of the ventilator 1100 is depicted in FIG. 13B, which illustrates a schematic depiction of an inhalation control feedback system 401 that determines an amount of gas flow 406 that is permitted to pass through an inhalation valve 411. The illustrated embodiment of the feedback system 401 is based on a target flow 421 and an actual flow 426 (or a flow within a line of the ventilator 1100). The position feedback may be used to determine flow, using the orifice characteristics of the valve and generally understood principles of fluid flow. Multiple gas types may be controlled based on the identified gas type (or gas id). The primary advantage of this flow measurement method is that the need for a separate flow sensor is eliminated and the resulting package provides for a compact flow delivery system.

As illustrated in FIG. 13B, a processor 431 receives an input signal relating to the actual flow 426 and compares the actual flow 426 to the target flow 421. Based on this comparison, the processor 431 sends a command signal 436 to an inhalation valve driver 441. The inhalation valve driver 441 is configured to control a position of the inhalation valve 411 to regulate the gas flow 406 through the inhalation valve 411. In the illustrated embodiment, the inhalation valve driver 441 sends a control current 446 to the inhalation valve 411 to maintain or adjust the inhalation valve 411 to modify or adjust the flow rate through the ventilator line.

For example, if the actual flow 426 was found to be too high, the processor 431 sends a command 436 to the inhalation valve driver 441 to close the inhalation valve 411 to reduce the flow rate through the ventilator line. The inhalation valve driver 441, upon receiving the command 436 to reduce the flow rate, adjusts the control current 446 to the inhalation valve 411 to decrease the opening of the inhalation valve 411 and reduce the flow rate within the ventilator line. As the control current 446 decreases the opening of the inhalation valve 411, the processor 431 receives position feedback 451 of the inhalation valve 411 via the inhalation valve driver 441, such that the processor 431 is able to determine the degree to which the inhalation valve 411 is open.

If the actual flow 426 input to the processor 431 was found to be too low, the processor 431 directs the inhalation driver 441 to adjust the control current 446 to the inhalation valve 411 to increase the opening of the inhalation valve 411 such that the flow rate through the ventilator line is increased. If the actual flow 426 input to the processor 431 was found to be at an acceptable level or within an acceptable range, the processor 431 directs the driver 441 to maintain the control current 446 to the inhalation valve 411 to maintain the position of the inhalation valve 411.

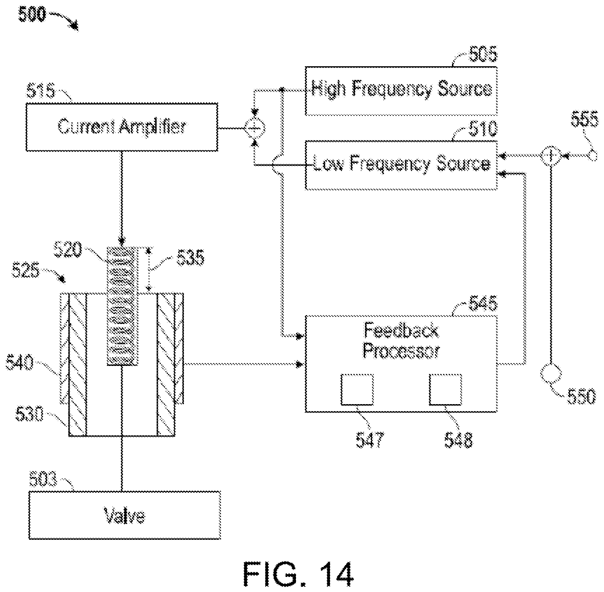

FIG. 14 illustrates an exemplary schematic arrangement of a current control system 500 that illustrates some embodiments of a driver (e.g., the exhalation valve driver 440 of FIG. 13A or the inhalation valve driver 441 of FIG. 13B) operating to adjust a valve 503 (e.g., the exhalation valve 410 or the inhalation valve 411). In the illustrated system 500, a high frequency source 505 generates a signal having a high frequency, and a low frequency source 510 generates a signal having a low frequency. The high frequency signal and the low frequency signal are summed together, and the signal is amplified by a current amplifier 515. In some embodiments, the current amplifier 515 is a linear current output amplifier. The signal is then directed to a coil 520 (e.g., a force coil) that is configured to move at least partly within a fixed magnetic field 525. The fixed magnetic field 525 is produced by a magnetic field generator, e.g., at least one permanent magnet 530 or a separate coil (not shown).

The natural frequency of the coil 520 is such that the coil 520 responds to the low frequency component of the combined signal by movement within or in relation to the magnetic field, as illustrated by arrows 535. In some embodiments, the low frequency component is less than about 90% of the natural frequency of the coil 520. In some embodiments, the low frequency component is less than about 80% of the natural frequency of the coil 520, and in yet further embodiments, the low frequency component is less than about 50% of the natural frequency of the coil 520.

The high frequency component of the combined signal preferably has a negligible effect on the position of the coil 520 such that the position of the coil 520 within the magnetic field is controlled substantially by the low frequency component. For example, in some embodiments, the high frequency component is more than 50% greater than the natural frequency of the coil 520. In some embodiments, the high frequency component can be between 50% and about 200% greater than the natural frequency of the coil 520. In yet additional embodiments, the high frequency can be more than 200% greater than the natural frequency of the coil 520.

A detection coil 540, or a feedback coil, detects the high frequency component of the signal passing through the coil 520, and the detection coil 540 sends a signal to a high frequency feedback processor 545 that determines, based on the detection coil 540 signal, a position of the coil 520 within the magnetic field 525. In some embodiments, a magnitude of the high frequency signal detected by the detection coil 540 is used to determine the position of the coil 520 within the magnetic field 525. In some instances, the high frequency feedback processor 545 also determines a velocity of the coil 520 within the magnetic field 525 and the high frequency feedback processor 545 sends a signal to the low frequency source 510 for providing feedback on the position and/or velocity of the coil 520. In some embodiments, the high frequency feedback processor 545 includes a position circuit 547 and a velocity circuit 548.

The low frequency source 510 also receives input from a sensor (not shown) within a ventilator line relating to how an actual condition 550 (e.g., pressure or flow rate) within the ventilator line compares to a target condition 555 of the ventilator line. Based on (i) the input relating to the comparison of actual condition 550 and the target condition 555 and (ii) the input from the high frequency feedback processor 545 relating to the position of the coil 520 in relation to the magnetic field 525, the low frequency source 510 determines whether the low frequency signal should be modified to change the position of the coil 520 in relation to the magnetic field 525.

For example, if the actual condition 550 were determined to be outside of an acceptable range of values set by the target condition 555, the low frequency source 510 changes the low frequency signal to move the coil 520 within the magnetic field 525. The coil 520 is preferably coupled, directly (e.g., mechanically) or indirectly (e.g., magnetically), to a portion of the valve 503 that regulates flow through the valve 503. Accordingly, movement of the coil 520 moves the portion of the valve 503 and changes an amount of gas passing through the valve 503. As the amount of gas passing through the valve 503 changes, the detected condition within the ventilator line changes, and the actual condition 550 is detected and compared with the target condition 555.

In some embodiments, it is advantageous to maintain a positive pressure within the ventilator line. For example, when the ventilator line is an exhalation line, or exhalation pathway, from a patient, and it is desirable to maintain a positive pressure within the patient's lungs relative to a local atmospheric pressure (or ambient pressure), the target condition 555 may include a minimum threshold pressure. When the actual condition 550 is determined to drop below the threshold pressure, the low frequency source 510 may be configured to close the valve 503, such that substantially no gas from the exhalation line passes through the valve 503. The valve 503, in such instances, may remain closed until the actual condition 550 within the exhalation line increases above the threshold pressure, at which time, the low frequency source 510 receives inputs reflecting that the valve 503 should be opened, and the source 510 changes the low frequency signal to move the coil 520 to a position in relation to the magnetic field 525 that corresponds to an opening of the valve 503. In some instances, upon receiving a signal that the actual condition 550 is above the threshold pressure, the low frequency source 510 may produce a signal that maintains position of the coil 520, and therefore the valve 503, to further increase the actual pressure within the exhalation line.

In some embodiments, it is advantageous to regulate a flow rate within the ventilator line. For example, when the ventilator line is an inhalation line, or inhalation pathway, to a patient, and it is desirable to regulate the flow rate to reach a target volume of gas, the target condition 555 may include a threshold time of flow rate. When the actual condition 550 is determined to reach the threshold time of flow rate, the low frequency source 510 may be configured to close the valve 503, such that substantially no gas from the inhalation line passes through the valve 503. The valve 503, in such instances, may remain closed until the next cycle, at which time, the low frequency source 510 receives inputs reflecting that the valve 503 should be opened, and the source 510 changes the low frequency signal to move the coil 520 to a position in relation to the magnetic field 525 that corresponds to an opening of the valve 503. In some instances, upon receiving a signal that the actual condition 550 has not reached the threshold time of flow rate, the low frequency source 510 may produce a signal that maintains position of the coil 520, and therefore the valve 503, to maintain the flow rate through the inhalation line.

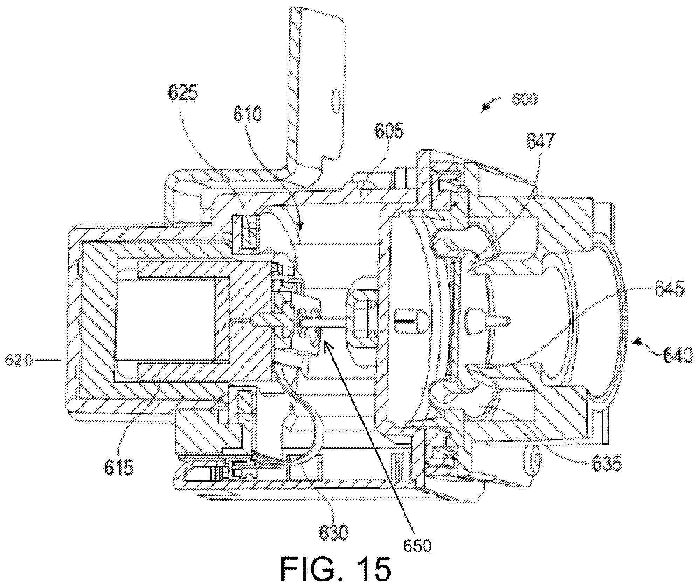

FIG. 15A is an exemplary cross sectional view of the a valve 600A, which may be the exhalation valve 410 or the inhalation valve 411, and operates under the same or similar principles described above with respect to valve 503 depicted in FIG. 14. The illustrated valve 600A includes a housing 605 that defines an internal chamber 610. Disposed within the internal chamber 610 is a coil 615 that is positioned and axially movable within or in relation to a fixed magnetic field generator 620. An armature 650 has a pole piece and may include or be attached to the coil 615. Positioned about at least a portion of the magnetic field generator 620 is a sensor 625. In some embodiments, the sensor 625 is a detection coil that is configured to detect high frequency signals passing through the coil 615. The high frequency signals detected by the sensor 625 are used to determine a position of the coil 615 within or in relation to the magnetic field generator 620.

A signal is communicated from the sensor 625 regarding a position of the coil 615, and signals are directed to the coil 615 via a flexible communication cable 630. As the signals directed to the coil 615 cause the coil 615 to move within the internal chamber 610 in relation to the magnetic field, movement of the coil 615 affects positioning of a convoluted diaphragm 635 and poppet 647 or seal. The poppet 647 operates as a variable orifice of the valve 600. Positioning of the poppet 647 with respect to the seat 645 affects the amount of fluid that passes through a valve having an opening 640.

Movement of the coil 615 can change a position of the sensor 625 by being directly coupled to the poppet 647 and moving the poppet 647 toward or away from a seat 645, which defines the valve orifice as the gap between the poppet 647 and seat 645. For example, the armature 650 may be directly connected to the diaphragm 635 and/or the poppet 647. In some embodiments, movement of the coil 615 can change a position of the poppet 647 by being indirectly coupled to the poppet 647. For example, a portion of the coil 615 and a portion of the poppet 647 may be magnetically opposed or attracted to each other. In such embodiments, movement of the coil 615 thereby opposes or attracts the portion of the poppet 647. In a similar configuration to direct coupling, this indirect coupling can affect positioning of the poppet 647 in connection with the seat 645 of the valve without contact between the coil 615 and the poppet 647.

Although a diaphragm with a poppet are illustrated in FIG. 15A, other types of valve configurations may be used in connection with the described embodiments. For example, other valves that can be used include, but are not limited to, a flap valve, a rotating disk valve, a duck-billed valve, etc.

The valve 600A can also provide increased stability by damping the moving components of the valve 600A. As explained above, a velocity of the coil 615 can be determined by a processor (e.g., processor 430 or 431 or high frequency feedback processor 545), which can include a velocity circuit that calculates a change of position with respect to time. The velocity can then be used to determine the desired damping. With the assumption that the valve 600A functions as a second order system, the damped frequency response is greater than or equal to about 40 Hz, and the damping coefficient that yields an under-damped or critically damped valve assembly. In other embodiments, additional damping such as pneumatic viscous damping can be incorporated into the valve 600A to further tune the valve 600 to the specific application.

The valve 600A can include a "fail-safe" open feature in case of loss of electrical power, software control, or loss of all inlet gases. The valve 600A can also be configured to switch to the "fail-safe" open configuration when the ventilator 1100 is turned off. On successful completion of power on checks, the ventilator 1100 will close the valve 600A and normal ventilation can commence. During a ventilator 1100 "fail-safe" open condition, the valve 600A, and other valves or ports will work in conjunction to (i) relieve pressure from the circuit down to ambient pressure conditions, (ii) allow ambient air to be available to the patient for breathing, and (iii) minimize re-breathing of gases.

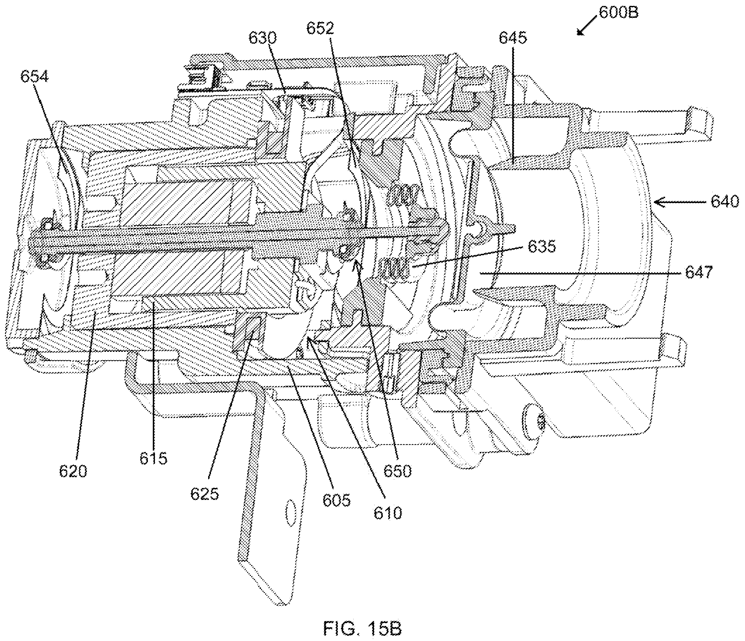

FIG. 15B illustrates a valve 600B, which may be another implementation of the valve 600A. The valve 600B may comprise similar components as the valve 600A. In addition, the valve 600B comprises a front flat spring 652, and a rear flat spring 654. The front flat spring 652 and the rear flat spring 654 provide mechanical or structural support for the armature 650. In other implementations, the armature 650 may be supported by other structures, such as bearings.

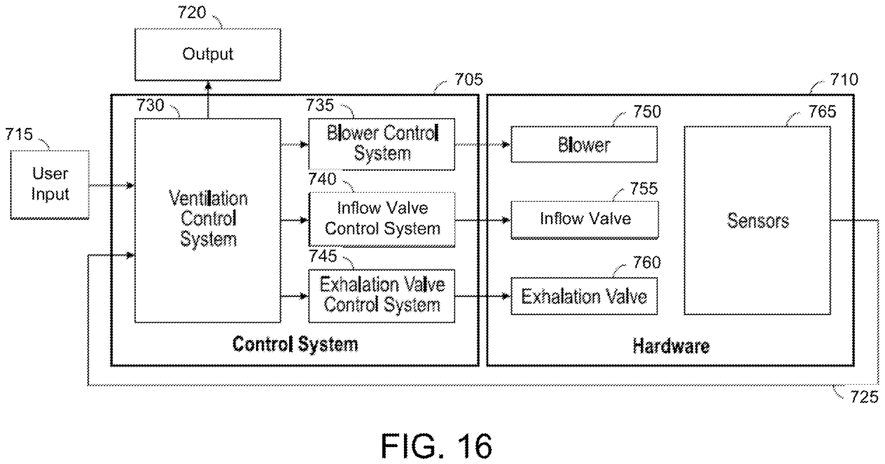

FIG. 16 illustrates a schematic depiction of another implementation of the ventilator 1100 having a control system 705, system hardware 710, user input 715, output 720, and feedback 725. The control system 705 includes a ventilation control system 730 that receives user input 715. The control system 705 includes hardware control systems that control respective hardware components of the ventilator 100. For example, the hardware control systems may include a blower control system 735, an inflow valve control system 740, and an exhalation valve control system 745. The blower control system 735 controls a respective blower 750, the inflow valve control system 740 controls a respective inflow valve 755, and the exhalation valve control system 745 controls a respective exhalation valve 760. The blower 750 may correspond to the blower 100 described herein and may be in fluid communication with the inflow valve 755 and/or the exhalation valve 760. For example, the blower 750 may be before or after the inflow valve 755 or the exhalation valve 760 in a fluid path of the ventilator 1100.

The system hardware 710 includes sensors 765 that detect information from the system hardware 710, for example, the blower 750, the inflow valve 755, and the exhalation valve 760. The sensors 765 produce one or more feedback signals 725 that are received by the ventilation control system 730. The ventilation control system 730 receives the feedback control signals 725 and the user input 715 and sends information to an output 720. The output 720 can include, for example, monitoring information and alarms. The feedback control signals 725 may also be used to provide inputs to the blower control system 735, the flow cassette control system 740, and the exhalation valve control system 745.

The inflow valve control system 740 may be similar to and operate similarly to the exhalation valve control system 745, which may correspond to the feedback system 400 in FIG. 13 or the current control system 500 in FIG. 14. The inflow valve 755 may also be similar to and operate similarly to the exhalation valve 760, which may correspond to the exhalation valve 410 in FIGS. 13 and 15, or the valve 503 in FIG. 14. Although labeled as inflow valve 755, the inflow valve 755 may be any front end valve before the patient in a gas flow. The exhalation valve 760 may be any back end valve behind the patient in a gas flow.

In FIG. 12, a flow cassette is used, whereas in FIG. 16, a valve control system is used instead. A flow cassette may include a pressure measurement device for an inlet gas, which measures pressure differential to determine flow measurement. The flow cassette may also include another valve tracker that drives the flow control valve of the flow cassette. Thus, a flow cassette provides flow measurement and flow control.

The valve control systems described herein provide flow control through the variable valve opening, but also provide flow measurement. The flow measurement can be derived from the position of the force coil or drive coil. Thus, the valve control systems also provide flow measurement and flow control, similar to flow cassettes. However, flow cassettes may be cost prohibitive for certain applications. For example, in certain applications, a ventilator system with valve control systems may be less expensive to produce than a ventilator system with one or more flow cassettes. The valve control systems may be different sizes, for example one quarter of the size of the other, as needed. The two valve control systems can work together, with one for inspiration and one for exhalation. For example, the inflow valve 755 may be open and regulated until an appropriate volume of gas has flowed to the patient. The inflow valve 755 will then close, and the exhalation valve 760 will open, and regulated until an appropriate volume of gas has been exhaled by the patient.

More particularly, gas is connected to the inflow valve 755 which starts closed, building up high pressure. The inflow valve control system 740 commands the inflow valve 755 to open, allowing the flow through to the patient. When inspiration starts, the exhalation valve 760 is closed. The inflow valve control system 740 determines when to close the inflow valve 755 based on a flow control or a pressure control. When the inflow valve 755 is closed, the exhalation valve control system 745 commands the exhalation valve 760 to open, allowing the patient to breathe out. The inflow valve 755 is directed to open, and the cycle repeats. Flow control may be calculated by sampling, for instance, the pressure every millisecond to make adjustments. Based on the position of the drive coil, the pressure can be calculated. The pressure is continuously monitored to adjust the position of the drive coil until a target flow is reached. The calculations may factor in ambient pressure, gas composition, gas temperature changes, downstream pressure changes, inlet pressure changes, etc. The calculations may further correct for standard conditions. By continuously monitoring pressure and adjusting the position of the drive coil, the exhalation valve 760 allows the patient to exhale without diffculty.

Although the flow control devices described herein may be used in connection CPAP therapy, other embodiments, particularly embodiments used on the front end of the ventilator, are not limited to CPAP therapy. The flow control devices described herein may be utilized at any point along a flow path of a ventilator, respirator, or other similar device. In addition, the flow control devices may be used in other fluid devices, particularly fluid devices which measure and/or regulate fluid flow, and are not limited to respiration.

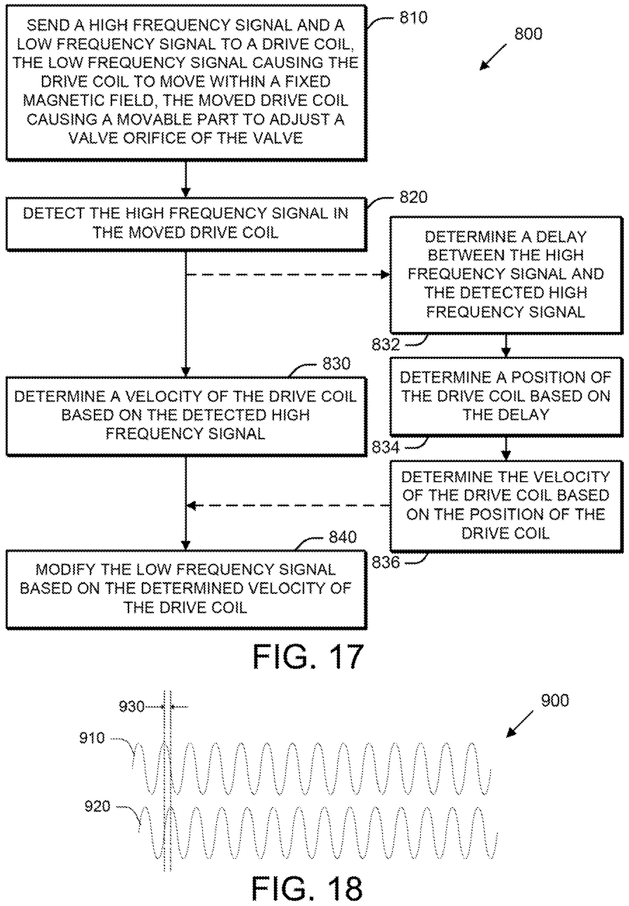

FIG. 17 shows a flowchart 800 of controlling a flow valve, such as the valve 503. At block 810, a high frequency signal and a low frequency signal is sent to a drive coil, such as the coil 615. The low frequency signal causes the drive coil to move within a fixed magnetic field, such as the fixed magnetic field generator 620. The moved drive coil causes a movable part, such as the poppet 647 or seal, to adjust a valve orifice of the valve, such as the opening 640. At block 820, the high frequency signal in the moved drive coil is detected. At block 830, a velocity of the drive coil is determined based on the detected high frequency signal. At block 840, the low frequency signal is modified based on the determined velocity of the drive coil. For example, the velocity signal may be injected into the low frequency source for the purpose of dampening.

The block 830 may be expanded into several operations, denoted by the dotted lines in FIG. 17. At block 832, a delay between the high frequency signal and the detected high frequency signal may be determined. FIG. 18 shows a sample space 900. A high frequency signal 910, which may be a high frequency current from the high frequency source 505, is compared to a detected high frequency signal 920, which may be a high frequency current detected in the drive coil after the drive coil moves. A delay 930 between the signals may be proportional to the position of the drive coil. Thus, at block 834, the position of the drive coil is determined based on the delay. At block 836, the velocity of the drive coil is determined based on the position of the drive coil. With the velocity determined at block 836, at block 840, the low frequency signal may be modified based on the determined velocity of the drive coil to, for example, control dampening of the drive coil.

A ventilator such as the ventilator 1100 may utilize an adapter described herein to facilitate connections.

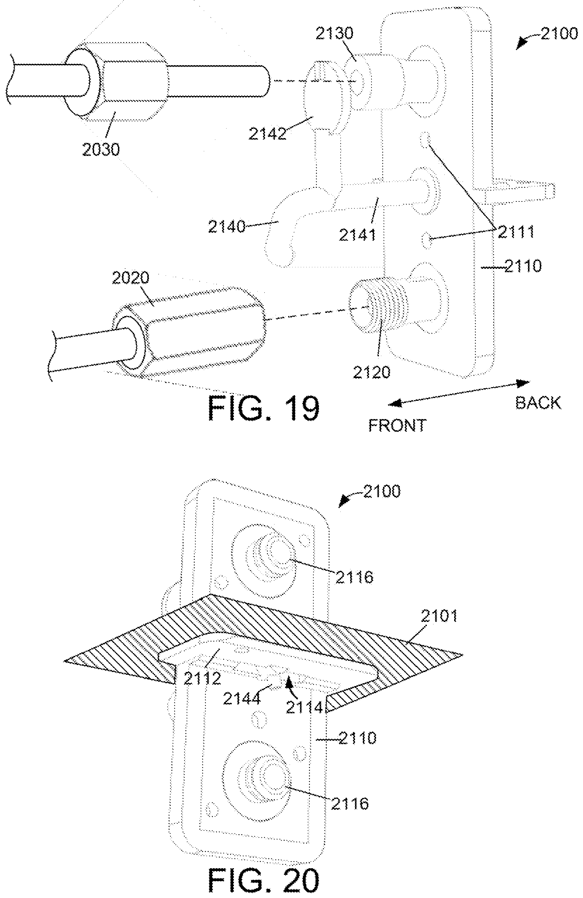

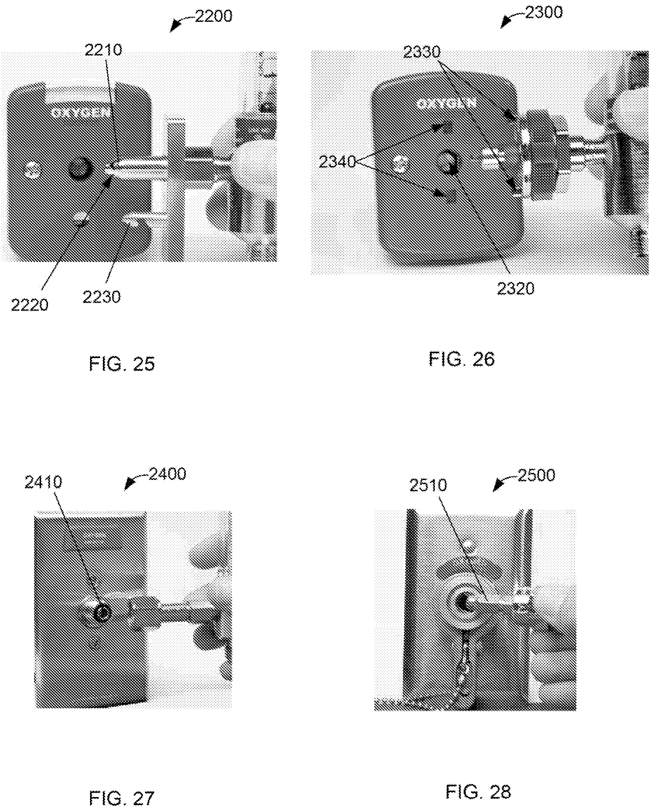

FIGS. 19-20 are front and back perspective views of an exemplary fluid inlet adapter 2100 according to certain aspects of the present disclosure. In FIG. 19, the fluid inlet adapter 2100, also referred to herein as "the adapter 2100," comprises a body 2110 with two inlets 2120, 2130 that are configured to respectively mate with connectors 2020, 2030 that are connected to two different fluid sources. In certain embodiments, the two connectors 2020 and 2030 may comprise different configurations comprising attributes such as shape, the presence or absence of thread, keys, etc. Example connector configurations are shown in FIGS. 25-28. A handle 2140 is movably coupled to the body 2110 and comprising an access control element 2142. In certain embodiments, the access control element 2142 is a paddle extending from a shaft 2141 that is, in this example, perpendicular to the body 2110. The handle 2140 is shown in FIG. 19 in a latched position, wherein the access control element 2142 is positioned in front of inlet 2130, thereby preventing a user from connecting a connector 2030 to the inlet 2130. In certain embodiments, the access control element 2142 is disposed in front of the inlet 2130. In certain embodiments, the access control element 2142 is disposed proximate to the inlet 2130, e.g. adjacent to the side of the inlet 2130, so as to interfere with the attachment of a connector 2030 to the inlet 2130 and substantially prevents connection to the inlet 2130 when the adapter 2100 is in the position shown in FIG. 19. The inlet 2120 is fully accessible in this position of the adapter 2100 and a user may connect a connector 2020 to the inlet 2120. The body 2110 may include one or more keying holes 2111 that engage pins, posts, or other keying features (not shown in FIG. 19) of the connectors 2020,2030.

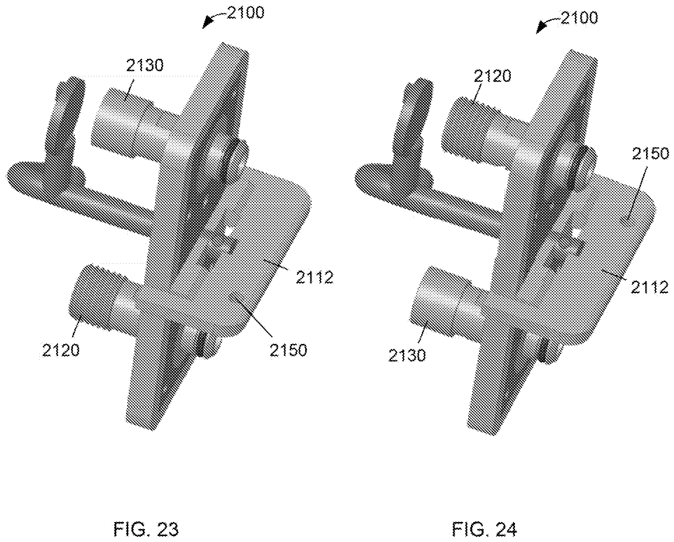

FIG. 20 depicts the back of the adapter 2100. A center plane 2101 is defined relative to the body 2110 and bisects the body 2110. There is an alignment feature 2112 extending from the body 2110 that is centered on the center plane 2101. There are two ports 2116 that are identical in form that are coupled to the body 2110 and symmetrically disposed on opposite sides of the center plane 2101. The adapter 2100 has a first position, as shown in FIG. 20, and a second position that is rotated 180° from the first position with respect to the plane of symmetry. Positions of the adapter 2100 are discussed in greater detail with respect to FIGS. 23 and 24. The adapter 2100 also comprises first and second coupling ports 2116 that are symmetrically located on opposite sides of the center plane 2101 on a back side of the body 2110. The first and second coupling ports 2116 are in respective fluid communication with the first and second inlets 2120, 2130. In certain embodiments, the coupling ports 2116 may be respectively aligned with the first and second inlets 2120, 2130. In certain embodiments, the coupling ports 2116 may be respectively offset from the first and second inlets 2120, 2130.

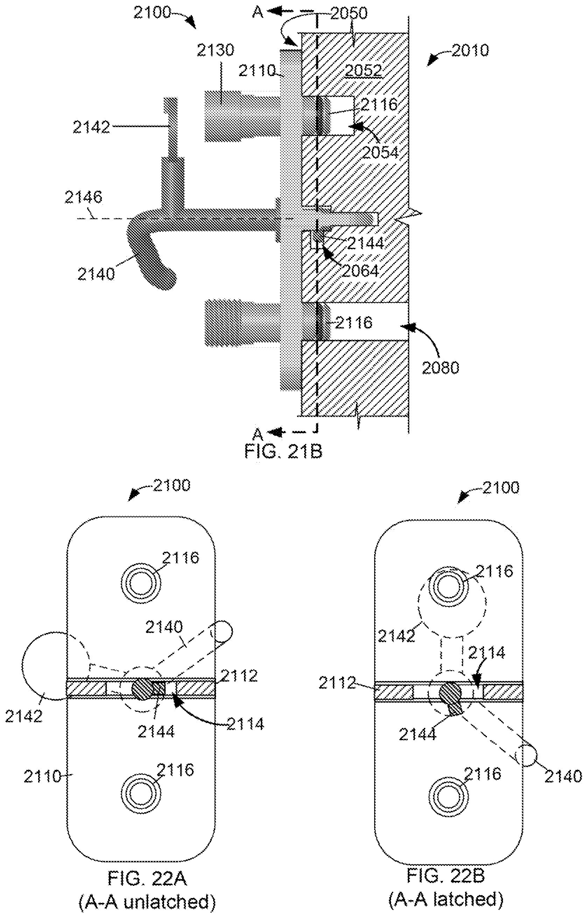

It can be seen in FIG. 20 that the handle 2140 comprises a latching pin 2144 that is disposed within a securing feature, for example slot 2114 formed in the alignment feature 2112. The latching pin 2144, in this example, extends outward from the portion of the shaft 2141 that extends beyond the bottom of the body 2110. The function of the pin latching 2144 and the method by which the handle 2140 secures the adapter 2100 to a device, for example a ventilator (not shown), is discussed in greater detail with respect to FIGS. 22A-22B.

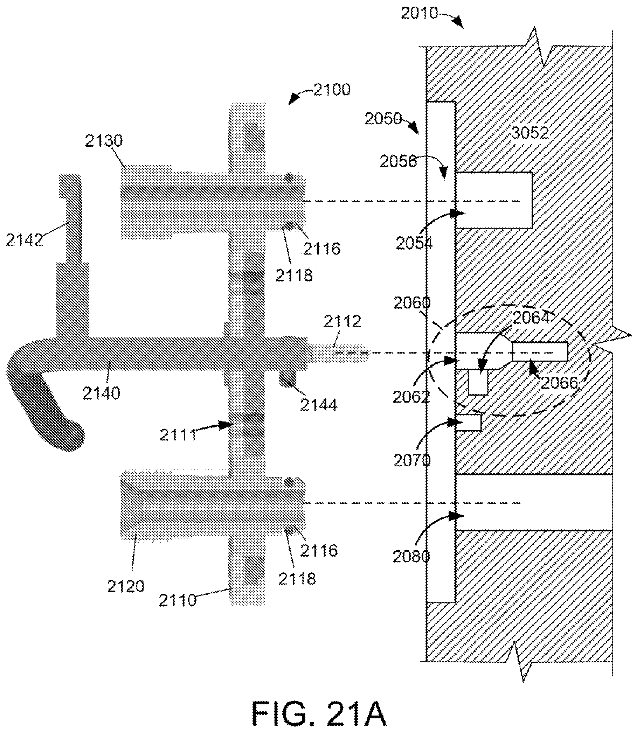

FIG. 21A is a cross-sectional side view of the exemplary fluid inlet adapter 2100 and a device 2010 according to certain aspects of the present disclosure. The device 2010 has a housing 2052 with, in this example, a docking station 2050 having an alignment slot 2066 that is configured to accept the alignment feature 2112. In this example, there is a recess 2062 adjacent to the alignment slot 2066 that is configured to accept an end of the handle 2140. A latching slot 2064 extends laterally from the recess 2062 and is configured to engage the pin 2144 when the handle 2140 is rotated such that the pin extends from the alignment feature 2112, as is discussed in greater detail with respect to FIGS. 22A-22B.

The housing 2052 comprises a fluid passage 2080 is configured to accept a flow of a fluid. In certain embodiments, the device 2010 is a ventilator and the fluid passage 2080 connects to a blower (not shown) that pumps the fluid from fluid passage 2080 to a patient as is generally known to those of skill in the art and not repeated herein. The fluid passage 2080 is positioned relative to the alignment slot 2066 such that one of the coupling ports 2116 will be at least partially disposed within the fluid passage 2080 when the adapter 2100 is secured to the device 2010 in either a first or second position. FIG. 21A depicts the example adapter 2100 secured to the docking station 2050 in the first position, wherein the coupling port 2116 that is in fluid communication with inlet 2120 is also at least partially disposed within and in fluid communication with the fluid passage 2080. In the second position (not shown in FIG. 21A), the adapter 2100 is upside down from the position shown in FIG. 21A such that the coupling port 2116 that is in fluid communication with inlet 2130 is also at least partially disposed within and in fluid communication with the fluid passage 2080. In certain embodiments, the coupling ports 2116 may have a sealing feature 2118, for example an O-ring, which is configured to detachably and sealingly mate with the fluid passage 2080. The housing also comprises a blind recess 2054 that accepts the un-used coupling port 2116. The docking location 2050 may have a recess 2056 configured to accept the body 2110 such that the front of the body 2110 is flush with the surface of the housing 2052. In certain embodiments, the docking location 2050 may also have a recess 2070 position under a keying hole 2111. The recess 2070 may provide clearance for a keying feature of a mating connector or may provide a retention function.

FIG. 21B is a cross-sectional side view of the exemplary adapter 2100 of FIG. 21A mated with the docking location 2050 of the housing 2052 according to certain aspects of the present disclosure. It can be seen that the lower coupling port 2116 is partially disposed within the fluid passage 2080 and the upper coupling port 2116 is partially disposed within the blind recess 2054.

FIGS. 22A-22B depict the position of the handle 2140 in exemplary unlatched and latched positions according to certain aspects of the present disclosure. FIG. 22A depicts the position of the handle 2140 while in an "unlatched" position suitable for insertion of the alignment feature 2112 into the alignment slot 2066 of the docking station 2050. The pin 2144 is positioned completely within the slot 2114 so as not to interfere with the alignment slot 2066. Once the adapter 2100 is fully seated in the docking station 2050, the handle 2140 can be turned to the position shown in FIG. 22B.

FIG. 22B depicts a "latched" position with handle 2140 rotated so as to engage pin 2144 in latching slot 2064. In this position of handle 2140, the access control element 2142 is disposed in front of the inlet 2130 thereby obstructing access to the inlet 2130 so as to discourage connection of a connector 2030 to the inlet 2130 while the adapter 2100 is secured to the device 2010 in this position.

FIGS. 23 and 24 depict an exemplary inlet adapter 2100 configured to accept fluid from two different sources 2020, 2030 according to certain aspects of the present disclosure. FIG. 23 depicts the adapter 2100 configured to enable inlet 2120 to allow a connector 2020 (not shown in FIG. 23) while blocking connection to the inlet 2130. It can be seen that the machine-detectable indicator 2150 is positioned in a first position, e.g. on the near side of alignment feature 2112.

FIG. 24 depicts the adapter 2100 reversed in orientation and configured to allow inlet 2130 to accept a connector 2030 (not shown in FIG. 24) while blocking connection to the inlet 2120. It can be seen that when the adapter 2100 is disposed in this position, which is the reverse of the position of FIG. 23, that the machine-detectable indicator 2150 is positioned in a second position, *e.g.* on the far side of alignment feature 2112, that is also the reverse of FIG. 23.

With respect to the positions of the machine-detectable indicators 2150 in FIGS. 23 and 24, the device 2050 may have a first sensor (not shown in FIG. 23) positioned so as to detect the presence of the sensor in the position of FIG. 23 and a second sensor positioned so as to detect the presence of the sensor in the position of FIG. 24. The use of two sensors may provide a positive indication of the position of the adapter 2100 and, therefore, a positive indication of which gas is being provided.

FIGS. 25-28 depict example connector configurations according to certain aspects of the present disclosure. The adapter 2100 may comprise inlets that are configured to accept one of these types of connectors. FIG. 25 depicts an "Ohmeda style" gas connection 2200 wherein the gas-specific configuration of the connector is accomplished by one or more notches 2220 on the outlet face 2210 and a pin 2230 on the adaptor. The notches 2220 and pins 2230 may vary in position and/or size based on the gas required.

FIG. 26 depicts a "Chemetron style" gas connection 2300 wherein the gas-specific configuration of the connector is accomplished by the position and shape of the latching hole 2320 on the outlet face and alignment tabs 2330 that mate with recesses 2340. The latching hole 2320 will vary in position and shape based on the gas required.

FIG. 27 depicts a "Diameter Index Safety System (DISS) style" gas connection 2400 wherein the gas-specific configuration of the connector is accomplished by gas-specific threads disposed on a barrel 2410. The thread diameter and adaptor nipple size may vary based on the gas required.

FIG. 28 depicts a "Schrader style" gas connection 2500 wherein the gas-specific configuration of the connector is accomplished by geometric indexing, i.e. each gas has a unique shape and size of the barrel 2510.

It can be seen that the disclosed embodiments of the inlet adapter provide a reliable means of configuring a device, such as a ventilator, to accept only one of a possible variety of gases. While the disclosed embodiment of the adapter has two inlets and accepts gas through one inlet while blocking the other inlet, other embodiments of the adapter may have three or more inlets and may be configured to accept gas through more than one of the three or more inlets. In addition, the machine-detectable indicator that is disclosed as a magnet herein may be any machine-readable element, for example a barcode or 2D matrix positioned to be read by a camera or scanner when the adapter is configured in a certain position.

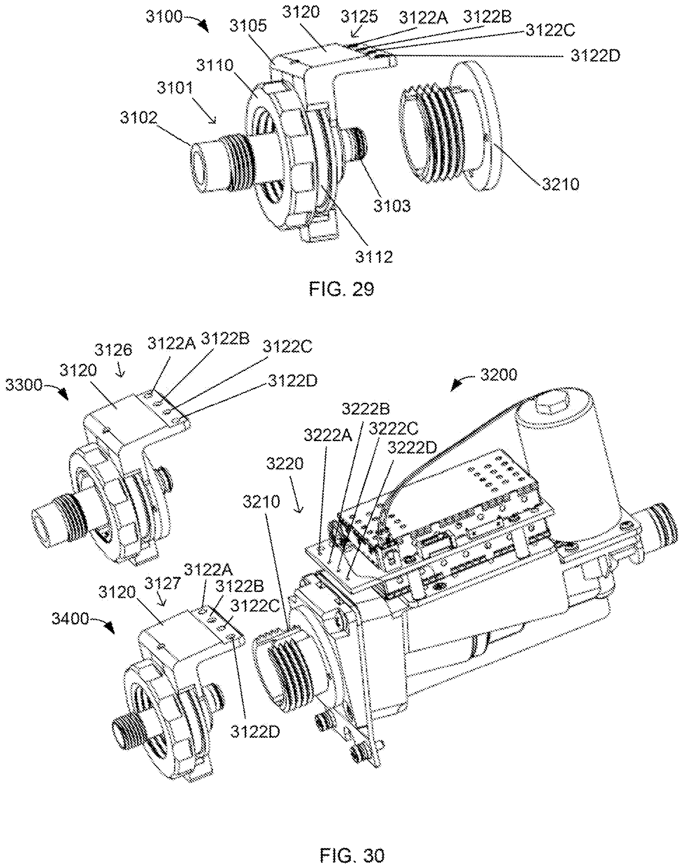

FIGS. 29-33 show implementations of adapters having one inlet. FIG. 29 shows an adapter 3100. The adapter 3100 has a housing 3105, an inlet 3101 extending through the housing 3105, a machine-detectable or machine-readable indicator 3120, and a latching component 3110. The inlet 3101 has a first end 3102 for connecting to a particular fluid source, and a connector 3103 for connecting to a flow control device 3200 (see in FIG. 30), which may be a flow cassette or other valve system. The latching component 3110, which may be a threaded nut that can be screwed by hand, is configured to interface with a threaded connector 3210 of the flow control device 3200. The latching component 3110 includes a groove 3112. The machine-readable indicator 3120 may be a tab which includes magnet positions 3122A, 3122B, 3122C, and 3122D, which may hold one or more magnets in a magnet configuration 3125. In some embodiments, one or more magnet positions 3122A, 3122B, 3122C, and 3122D may not hold any magnet. Although the magnet positions 3122A-D may be in a linear arrangement, in other implementations, other arrangements may be used.

FIG. 30 shows another adapter 3300. The adapter 3101 has a magnet configuration 3126, which may have magnets in magnet positions 3122B and 3122C. The magnets may be embedded within the machine-readable indicator 3120. Also seen in FIG. 30, an adapter 3400 has a magnet configuration 3127, which may have magnets in magnet positions 3122B and 3122D.

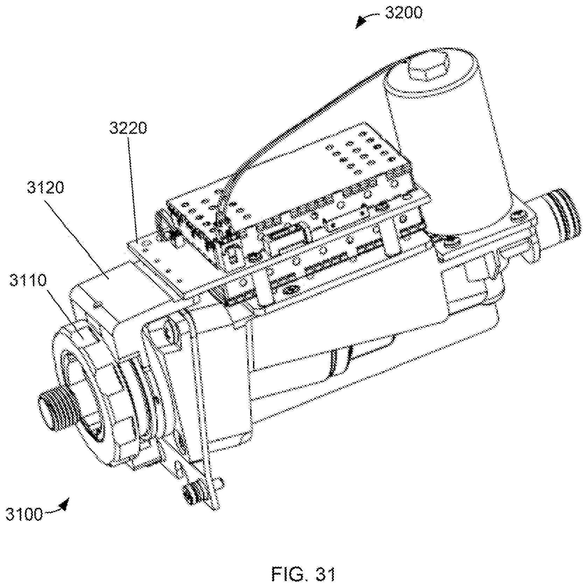

The magnet configurations may be detected by a sensor 3220 of the flow control device 3200. The sensor 3220 may include magnet sensors 3222A, 3222B, 3222C, and 3222D, which may correspond to the magnet positions 3122A-D. FIG. 31 shows the adapter 3100 connected to the flow control device 3200, which may be secured by tightening the latching component 3110. The sensor 3220 aligns with the machine-readable indicator 3120. The sensor 3220 is configured to detect the magnet configurations of adapters. The magnet configurations correspond to specific fluid sources. For example, the magnet configuration 3126 may correspond to heliox, and the magnet configuration 3127 may correspond to oxygen. The inlet 3101 may be configured for connection to the corresponding fluid source. By detecting the magnet configuration, the flow control device 3200 can identify the fluid passing through the flow control device 3200. After identifying the fluid, the various processors of the ventilator 1100 may use parameters corresponding to the identified fluid for further calculations as described herein.

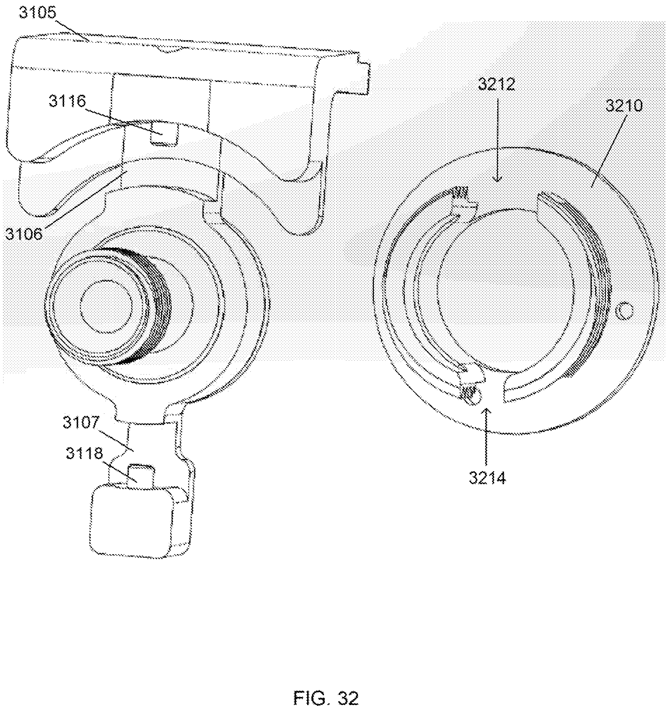

FIG. 32 shows the adapter 3100 without the latching component 3110. The housing 3105 includes a first protrusion 3106, and a second protrusion 3107. The housing 3105 also includes a first pin 3116, and a second pin 3118. The first and second pins 3116 and 3118 are configured to interface with the groove 3112 of the latching component 3110, allowing the latching component 3110 to rotate freely without being separated from the housing 3105. The threaded connector 3210 includes a first notch 3212 and a second notch 3214.

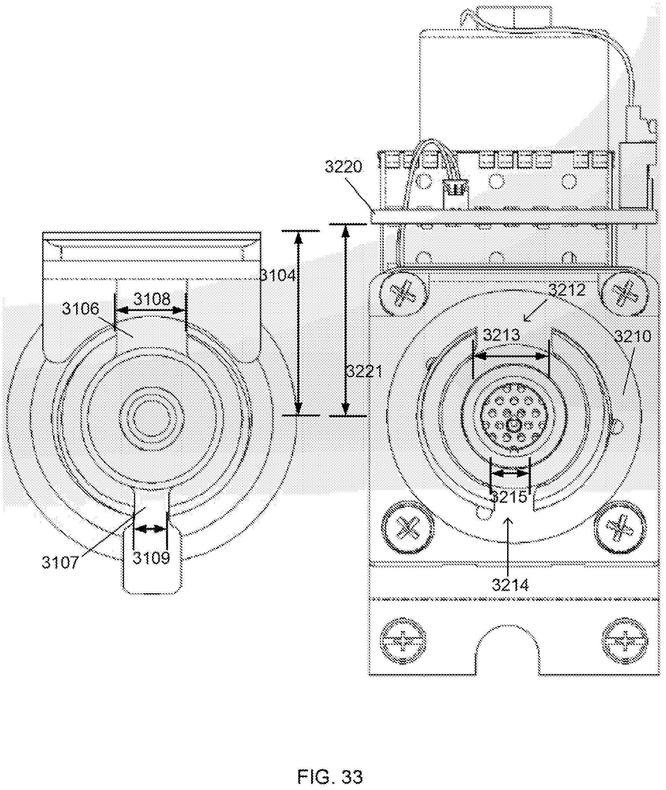

FIG. 33 further shows the adapter 3100 and the flow control device 3200. A first width 3108 of the first protrusion 3106 corresponds to a first width 3213 of the first notch 3212. A second width 3109 of the second protrusion 3107 corresponds to a second width 3215 of the second notch 3214. The first protrusion 3106 is configured to fit into the first notch 3212, and the second protrusion 3107 is configured to fit into the second notch 3214. Because the first and second widths are different, the adapter 3100 can only fit into the threaded connector 3210 in one orientation, to mitigate incorrect insertions. In addition, a distance 3104 between the inlet 3101 and the machine-readable indicator 3120 corresponds to a distance 3221 between the threaded connector 3210 and the sensor 3220 such that the sensor 3220 can detect the machine-readable indicator 3120. The machine-readable indicator 3120 may be properly aligned with the sensor 3220 when the latching component 3110 is fully tightened. When the machine-readable indicator 3120 is not properly detected by the sensor 3220, or the magnet configuration is unknown (e.g., a magnet is missing or in the wrong magnet position), an alarm condition may occur. In addition, when the adapter 3100 is not properly or fully connected, pressurized gas may leak, causing an audible notification to the operator.

For example, the connector 3103 may be configured to have a length such that the connector 3103 provides fluid communication with the flow control device 3200 at an intermediate portion along the threaded connector 3210's path when being connected. The length of the connector 3103 extends such that there may be fluid communication even when the latching component 3110 is not fully engaged. Upon removal of the adapter 3100, for instance by disengaging the latching component 3110, an audible hissing sound may inform the operator that the supply must be turned off and/or that the adapter 3100 should be fully removed from the source to limit complications associated with improper removal procedures.

The use of multiple adapters 3100 allow a single flow control device 3200 to connect to various fluid sources, rather than having a flow control device for each possible fluid source. Reducing the number of flow control devices may reduce the size of the ventilator. The multiple adapters may be tethered to the ventilator in order to prevent misplacement of the adapters.

The systems and methods described herein for measuring flow rates and compensating for the composition of the gas or gas mixture as well as the temperature of the measured gas provides increased accuracy compared to flow measurements made within conventional ventilators.

Turning to FIGS. 11A-B, a flow cassette 4200 may be installed within the housing 1110 behind the gas inlet adapter 1120 and in fluid communication between the inlet connector 1126 shown in FIG. 11B and the supply port 1155 shown in FIG. 11A.

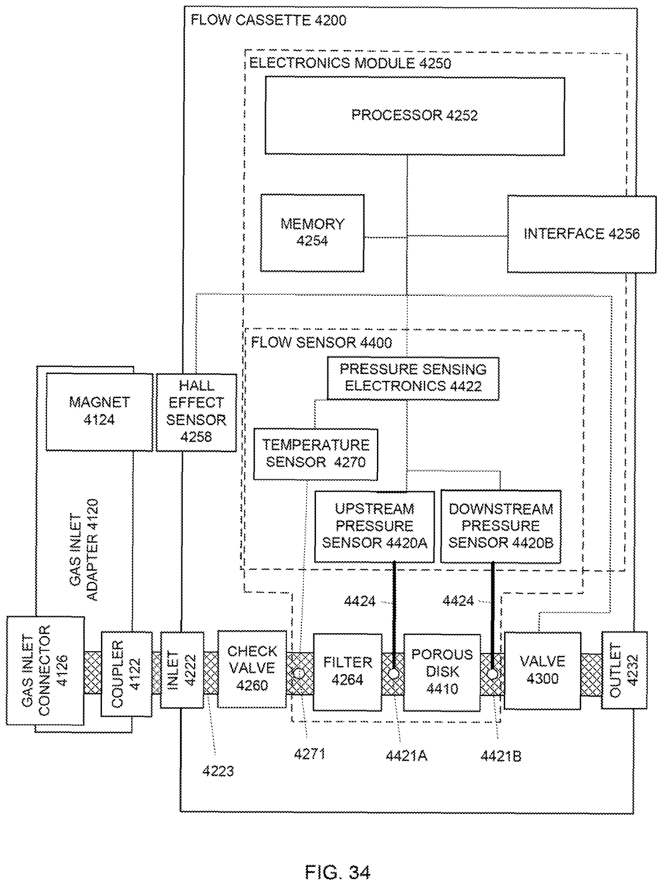

FIG. 34 is a block diagram of an exemplary flow cassette 4200 according to certain aspects of the present disclosure. The flow cassette 4200 may correspond to the flow cassette control system 340 described above. The flow cassette 4200 includes an inlet 4222 that is configured to sealingly mate with an input flow channel, for example a coupler 4122 of the gas inlet adapter 4120. The gas inlet adapter 4120 also has an inlet connector (not shown in FIG. 34) that is fluidly connected to the coupler 4122. Various breathing gases and gas mixtures are associated with individually unique connector types, sizes, and configurations, wherein the association is generally recognized in the medical industry. Each gas inlet adapter 4120 has one or more inlet connectors that are adapted to respectively accept a connector that is unique to a certain type of gas or gas mixture. The number and placement of magnets 4124 are uniquely associated with the inlet connector that will be coupled to the inlet of the flow cassette 4200 when that gas inlet adapter 4120 is installed in a ventilator and thereby mated with the flow cassette 4200. In certain embodiments, the gas inlet adapter 4120 may be configured to accept one or more of a standard composition of ambient air, a pure oxygen, and a heliox gas mixture.

The inlet 4222 is fluidly connected to a passage 4223 that runs through the flow cassette 4200 to an outlet 4232 that is configured to sealingly mate with an output flow channel of the ventilator 4100 that, for example, leads to the supply limb 4104. In this example embodiment, there are several elements disposed along the passage 4223, including a check valve 4260, a filter 4264, a porous disk 4410 and a valve 4300. In certain embodiments, some of these elements may be omitted or arranged in a different order along the passage 4223. In this embodiment, the flow cassette 4200 also includes a Hall effect sensor 4258 configured to detect the number and placement of the magnets 4124 of the gas inlet adapter 4120. By comparing the detected number and placement of the magnets 4124 to stored information associating the number and placement of the magnets 4124 with gases that will be accepted by the inlet connector that is coupled to the inlet of the flow cassette 4200, the processor 4252 can automatically determine what gas will be provided through the gas inlet adapter 4120 as installed in the ventilator 4100. In other embodiments, the gas inlet adapter 4120 may include another type of indicator, for example a machine-readable element, that is associated with the configuration of the gas inlet adapter 4120 and the flow cassette 4200 may include a sensor that is capable of reading the machine-readable element and thereby automatically detecting the configuration of the gas inlet adapter 4120.

The flow cassette 4200 includes a flow sensor 4400 that has a flow restriction 4410 that, in this example, is a porous disk disposed in passage 4223 such that all gas flowing through the passage 4223 must pass through the porous disk 4410. The flow sensor 4400 also includes an upstream pressure sensor 4420A and downstream pressure sensor 4420B with gas passages 4424 from the sensors to sensing ports 4421 A and 4421B disposed in the passage 4223 on upstream and downstream sides, respectively, of the porous disk 4410. There is also a temperature sensor 4270 that has a temperature sensing element 4271 disposed in the passage 4223. In conjunction with the knowledge of which gas is flowing through the porous disk 4410, derived from the configuration of the gas inlet adapter 4120 as indicated by the magnet 4128 and sensed by the Hall effect sensor 4258, and the knowledge of the temperature of the gas, as measured by the temperature sensor 4270, the pressure drop can be used to determine the true flow rate, sometimes referred to as "the compensated flow rate," of the gas that is passing through the porous disk 4410.

The pressure drop across the porous disk 4410 is related in a monotonic way to the rate of gas passing through the porous disk 4410. The porous disk 4410 is characterized as to its flow resistance characteristics with a selection of gases and gas mixtures at a standard temperature. Without being bound by theory, certain gases, such as helium, have a smaller molecular size and pass more easily through the thickness of the porous disk 4410 compared to a gas, such as nitrogen, with a larger molecule. Thus, a certain pressure drop will indicate a first flow rate for a small-molecule gas and a second, lower flow rate for a large-molecule gas. Gas mixtures will tend to have flow rates that reflect the percentage composition of the gases that make up the gas mixture. In certain embodiments, the pressure drops of certain predetermined medical gases and gas mixtures are specifically characterized for the porous disk 4410 and stored in a look-up table contained in the memory 4254 of the electronics module 4250. The temperature of a gas also affects the pressure drop for a given flow rate of that gas flowing through the porous disk 4410. In certain embodiments, the effect of the gas temperature is also characterized for the porous disk 4410 and stored in the memory 4254. In certain embodiments, the characterization of the flow characteristics of the porous disk 4410, also referred to herein as "compensation parameters," are combined for gas type and temperature in a single look-up table. Those of skill in the art will recognize that such compensation parameters may be stored in other forms, for example equations that include scaling parameters, to enable conversion of a raw pressure drop measurement into an accurate flow rate.

The flow cassette 4200 includes an electronics module 4250. In certain embodiments, the conversion of the raw pressure measurements by pressure sensors 4420A, 4420B into a pressure drop measurement is accomplished in a separate pressure sensing electronics 4422 and provided to a flow sensor processor 4252. In certain embodiments, the pressure sensing electronics 4422 may provide the processor 4252 with individual pressure signals for pressures that are upstream and downstream of the porous disk 4410. In certain embodiments, there may also be a front connection panel 4160 for connection to, for example, external instruments, sensors, or sensor modules. In certain embodiments, the pressure sensors 4420A, 4420B may provide the raw signals directly to the processor 4252. In certain embodiments, the pressure sensors 4420A, 4420B may include conversion circuitry such that each sensor 4420A, 4420B provides a pressure signal directly to the processor 4252.

In certain embodiments, the temperature sensor 4270 provides a signal that includes a temperature to the pressure sensing electronics 4422. In certain embodiments, the temperature sensor 4270 provides this temperature signal directly to the processor 4252. In certain embodiments, the temperature sensing element 4271 may be connected directly to the pressure sensing electronics 4422 or to the processor 4252. In certain embodiments, the temperature sensor 4270 may be configured to sense the gas temperature over a range of temperatures of at least 5-50° C. In certain embodiments, the temperature sensor 4270 may be configured to sense the gas temperature over a range of temperatures of at least 5-50° C.

The processor 4252 is connected to the memory 4254 and an interface module 4256 as well as the sensors 4270, 4420A, and 4420B. The various drive, sensing, and processing functions of these sensors 4270, 4420A, and 4420B may be accomplished in various different modules, such as the processor 4252 and pressure sensing electronics 4422, depending on the particular design and layout of the flow cassette 4250 without departing from the scope of this disclosure. For example, a processor 4252 may be configured to provide a supply an electrical current directly to the temperature sensing element 4271 and to directly measure a voltage drop across the temperature sensing element 4271 without the need for intervening electronics. All functions disclosed herein may be accomplished in the block elements of FIG. 34 as described or in alternate blocks and the blocks depicted in Fig. 34 may be combined or divided without departing from the scope of this disclosure.

The memory 4254 is configured to store operating instructions for the processor 4252 and data that may include calibration data for the sensors 4258, 4270, 4420A, and 4420B. The data may also include information, as discussed above, such as equations or look-up tables to use the two pressure measurements from pressure sensors 4420A and 4420B and the temperature measurement from the temperature sensor 4270 to determine a flow rate through the porous disk 4410. In certain embodiments, the memory comprises non-volatile memory such as magnetic disk, a solid-state memory, a flash memory, or other non-transient, non-volatile storage device as known to those of skill in the art.

The processor 4252 is also operatively coupled to the valve 4300 and is capable of actuating the valve 4300. The interconnection of the processor 4252 with the other elements as shown in FIG. 34 may be accomplished by direct connection via any technology known to those of skill in the art, for example twisted-pair wires or fiber-optic cables, or via a network connection with microprocessors embedded in the other elements. The interface module 4256 may include signal transceivers for wired or wireless communication with other devices within the ventilator 4100 or may connector to an external interface, such as the rear interface panel 1165 shown in FIG. 11B, to communicate with devices external to the ventilator 1100.

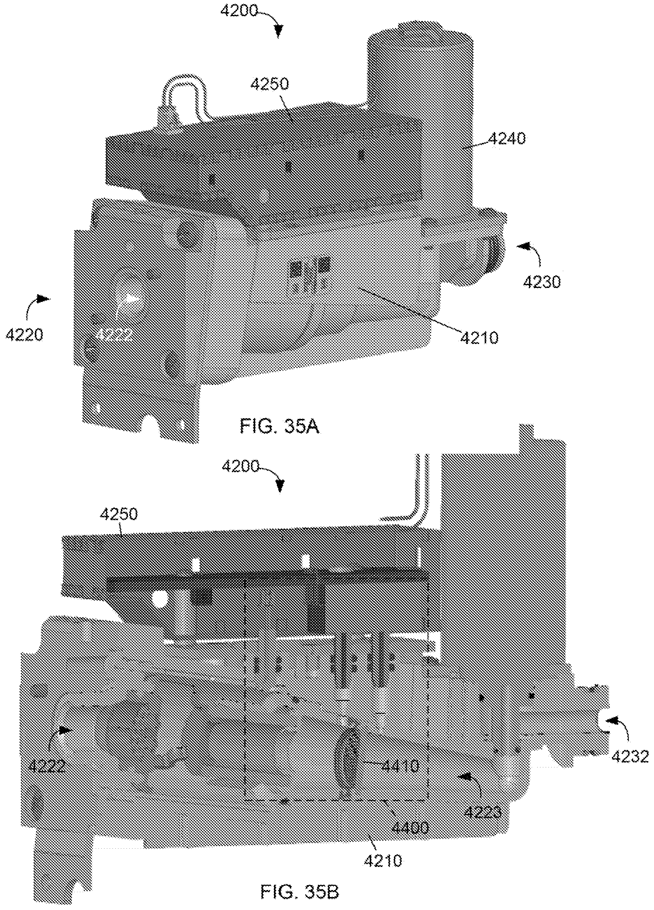

FIG. 35A depicts an exemplary flow cassette 4200 according to certain aspects of the present disclosure. The flow cassette 4200 has a body 4210 with an inlet end 4220 and an outlet end 4230. At the inlet end 4220, there is the inlet 4222 that is configured to sealingly mate with a coupler 4122 (not shown in FIG. 35A) of a gas inlet adapter 4120. The inlet end 4220 may also include locating features 4226, for example protruding pins, that align the gas inlet adapter 4120 to the inlet 4222 and a mating face 4224 that provides a reference surface for the mated gas inlet adapter 4120. A solenoid 4240 is attached to the body proximate to the outlet end 4230 to drive a pressure control valve (not visible in FIG. 35A) disposed within the body 4210. The electronics module 4250 is attached, in this embodiment, to the top of the body 4210. The details of the electronics module are discussed in greater detail with respect to FIG. 34.

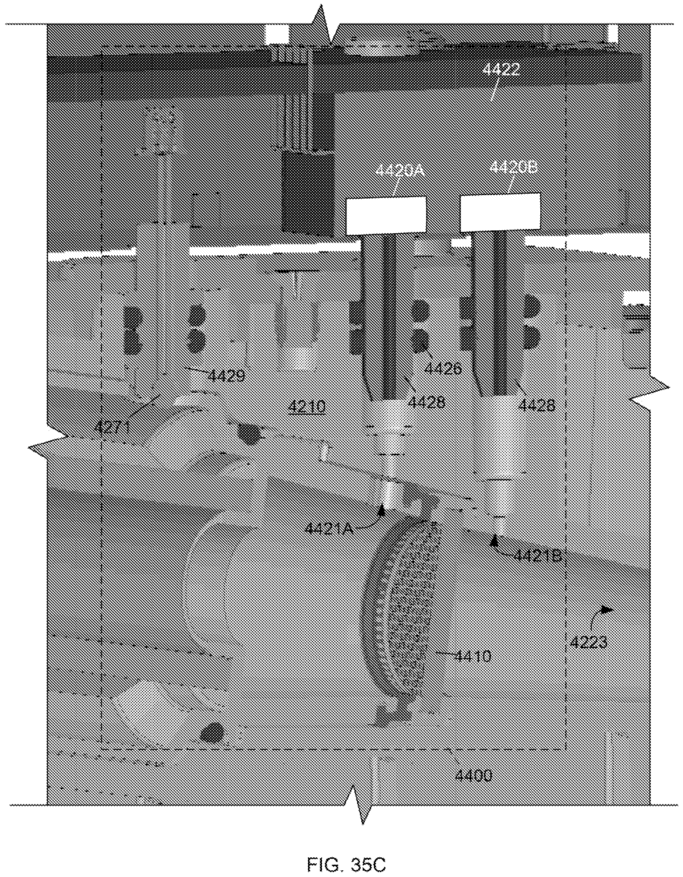

FIG. 35B is a cross-section of the flow cassette of FIG. 35A according to certain aspects of the present disclosure. The dashed-line box 4400 indicates the elements that make up the flow sensor 4400, which is discussed in greater detail with respect to FIGS 34 and 35C. The passage 4223 that connects the inlet 4222 and outlet 4232 is visible in the cross-section of FIG. 35B, with the porous disk 4410 disposed within the passage 4223.

FIG. 35C is an enlarged view of a portion of FIG. 35B showing the exemplary flow sensor 4400 according to certain aspects of the present disclosure. In this example, the pressure sensors 4420A, 4420B are disposed within the package of the pressure sensing electronics 4422 and connected to the passage 4223 by gas passages 4424 leading to sensing ports 4421 A and 4421B. The temperature sensing element 4271 is exposed to the interior of the passage 4223 and therefore in contact with the gas within the passage 4223. Seals 4426, in this example a pair of O-rings, provide a gas-tight seal between the housing 4210 and the tube extensions 4428 for the gas passages 4424 and a feed-through 4429 for the temperature sensing element 4271.

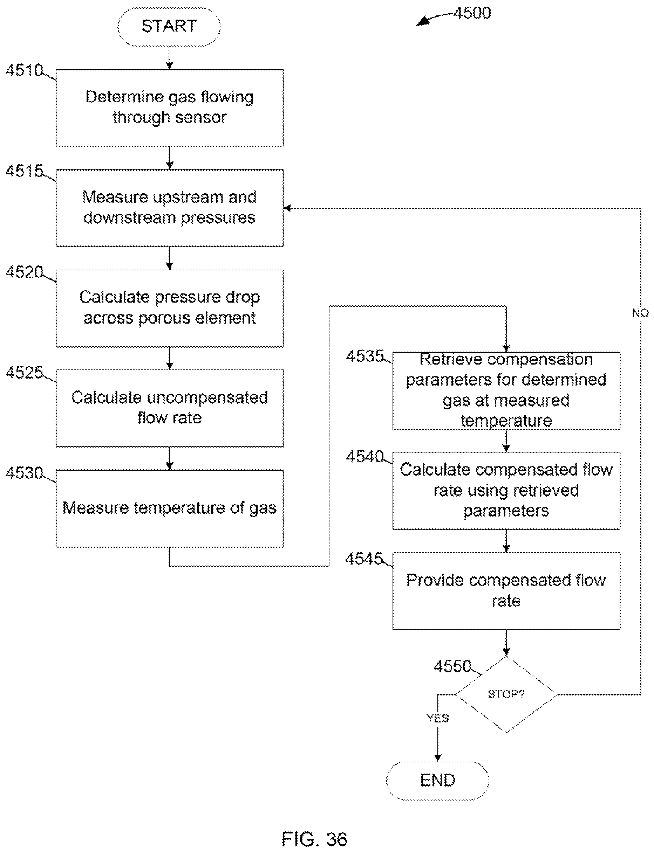

FIG. 36 is a flow chart of an exemplary flow measurement process 4500 according to certain aspects of the present disclosure. The process 4500 starts in step 4510 by determining which gas or gas mixture, for example oxygen or heliox 70/30, will be flowing through the flow sensor 4400. In step 4515, the gas pressures upstream and downstream of the flow restriction 4410 are measured and a pressure drop across the flow restriction 4410 is calculated in step 4520. In step 4525, the processor 4252 calculates an uncompensated flow rate based at least partially on the pressure drop. The temperature of the gas flowing through the flow sensor 4400 is measured in step 4530 and in step 4535 the processor 4252 loads information from the memory 4254 that may include compensation parameters related to the flow sensor 4400. The processor 4252 calculates a compensated flow rate using the retrieved compensation parameters in step 4540 and provides this compensated flow rate, for example to a processor of the ventilator 1100, in step 4545. Step 4550 is a decision point that checks whether a "stop" command has been received, in which case the process 4500 branches along the "yes" path to the end and terminates. If a "stop" command has not been received, the process 4500 branches along the "no" path back to step 4515 and measures the pressures and temperature. The process 4500 will loop through the steps 4515-4550 until a "stop" command is received.

In summary, it can be seen that the disclosed embodiments of the flow sensor provide an accurate measurement of a gas flow rate in a compact and modular form. The accuracy of the flow rate may be improved by compensating for one or more of the gas temperature and the gas composition. This compensation may be accomplished through prior experimental calibration of the particular flow restriction, e.g. porous disk, or calculations based on gas flow theory. The modular form enables this subsystem to be independently tested and calibrated as well as simplifying assembly and replacement.

It is advantageous to provide a modular flow cassette that provides accurate flow measurements of a variety of gases and gas mixtures over a range of temperatures and flow rates.

Turning to FIGS. 11A-B, flow cassette 5200, similar to flow cassette 4200, may be installed within the housing 1110 behind the gas inlet adapter 1120 and in fluid communication between the inlet connector 1126 shown in FIG. 11B and the supply port 1155 shown in FIG. 11A.

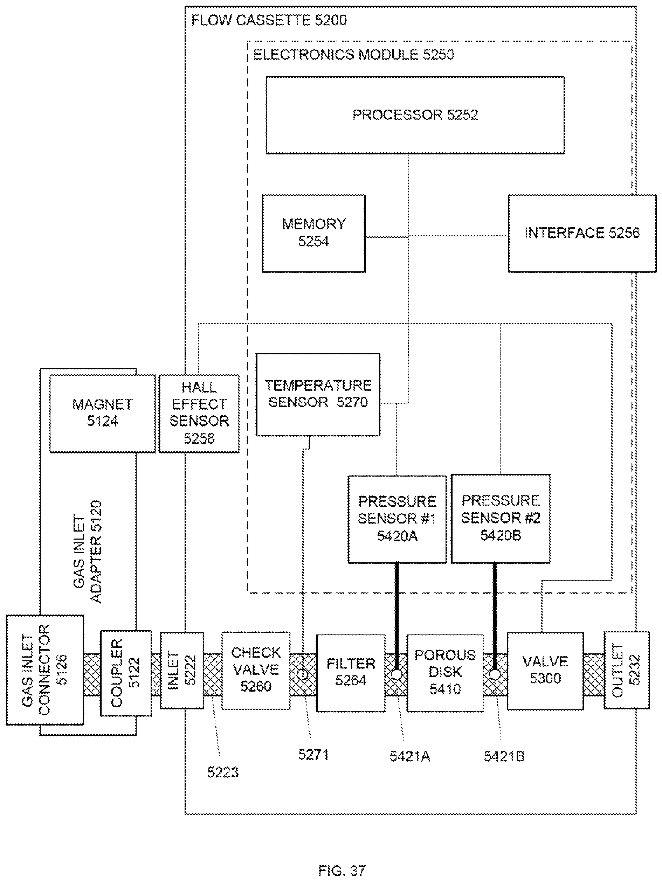

FIG. 37 is a block diagram of an exemplary flow cassette 5200 according to certain aspects of the present disclosure. The flow cassette 5200 includes an inlet 5222 that is configured to sealingly mate with an input flow channel, for example a coupler 5122 of the gas inlet adapter 5120. The gas inlet adapter 5120 also has an inlet connector 5126 that is fluidly connected to the coupler 5122. Various breathing gases and gas mixtures are associated with individually unique connector types, sizes, and configurations, wherein the association is generally recognized in the medical industry. Each gas inlet adapter 5120 has one or more inlet connectors 5126 that are adapted to respectively accept a connector that is unique to a certain type of gas or gas mixture. The gas inlet adapter 5120 may include one or more magnets 5124 wherein the number and placement of magnets 5124 are uniquely associated with the inlet connector 5126 that will be coupled to the inlet 5222 of the flow cassette 5200 when that gas inlet adapter 5120 is installed in a ventilator 1100 and thereby mated with the flow cassette 5200. In certain embodiments, the gas inlet adapter 5120 may be configured to accept one or more of a standard composition of ambient air, a pure oxygen, and a heliox gas mixture.

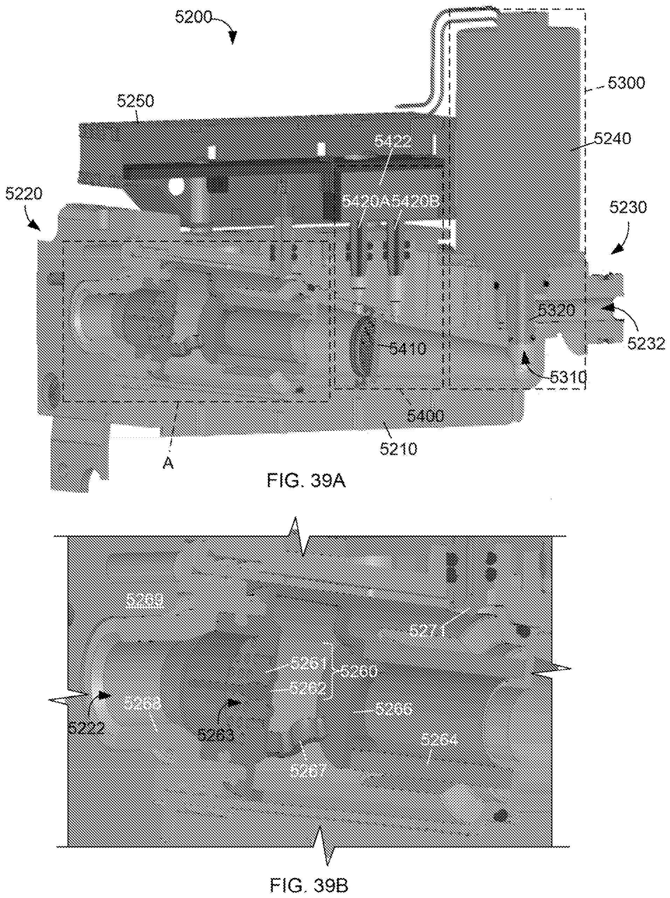

The inlet 5222 is fluidly connected to a passage 5223 that runs through the flow cassette 5200 to an outlet 5232 that is configured to sealingly mate with an output flow channel of the ventilator 1100 that, for example, leads to the supply limb 1104 in FIG. 10. In this example embodiment, there are several elements disposed along the passage 5223, including a check valve 5260, a filter 5264, a porous disk 5410 and a valve 5300. In certain embodiments, some of these elements may be omitted or arranged in a different order along the passage 5223. These elements are discussed in greater detail with respect to FIGS. 39A and 39B. In this embodiment, the flow cassette 5200 also includes a Hall Effect sensor 5258 configured to detect the number and placement of the magnets 5124 of the gas inlet adapter 5120. By comparing the detected number and placement of the magnets 5124 to stored information associating the number and placement of the magnets 5124 with gases that will be accepted by the inlet connector that is coupled to the inlet of the flow cassette 5200, the processor 5252 can automatically determine what gas will be provided through the gas inlet adapter 5120 as installed in the ventilator 1100. In other embodiments, the gas inlet adapter 5120 may include another type of indicator, for example a machine-readable element, that is associated with the configuration of the gas inlet adapter 5120 and the flow cassette 5200 may include a sensor that is capable of reading the machine-readable element and thereby automatically detecting the configuration of the gas inlet adapter 5120.

The flow cassette 5200 includes an electronics module 5250. In certain embodiments, the electronics module 5250 includes a temperature sensor 5270 that has a temperature sensing element 5271 disposed in the passage 5223. The electronics module 5250 also includes pressure sensors 5420A and 5420B that are respectively connected through passages to ports 5421 A and 5421B in the passage 5223 that are disposed on opposite sides of the porous disk 5410.

The electronics module 5250 also includes a flow cassette processor 5252 that is connected to a memory 5254 and an interface module 5256. The processor 5252 is also coupled to the sensors 5258, 5270, 5420A and 5420B and is configured to receive signals from each sensor that are associated with the measured parameter of each respective sensor. The memory 5254 is configured to store operating instructions for the processor 5252 and data that may include calibration data for the sensors 5258, 5270, 5420A, and 5420B. The data may also include information such as equations or look-up tables to use the two pressure measurements from pressure sensors 5420A and 5420B to determine a flow rate through the porous disk 5410. In certain embodiments, additional sensors, e.g., a barometric pressure transducer, outside the ventilator 1100 may be used to correct the measured flow for surrounding conditions. The processor 5252 is also operatively coupled to the proportional valve 5300 and is capable of actuating the valve 5300. The interconnection of the processor 5252 with the other elements as shown in FIG. 37 may be accomplished by direct connection via any technology known to those of skill in the art, for example twisted-pair wires or fiber-optic cables, or via a network connection with microprocessors embedded in the other elements. The interface module 5256 may include signal transceivers for wired or wireless communication with other devices within the ventilator 1100, for example a central processor (not shown in FIG. 37), or may connect to an external interface, such as the rear interface panel 5165 shown in FIG. 11B, to communicate with devices external to the ventilator 1100. Interface 5256 may be configured to accept both power and communication signals and, in certain embodiments, may include one or more voltage converters to provide power to the module.

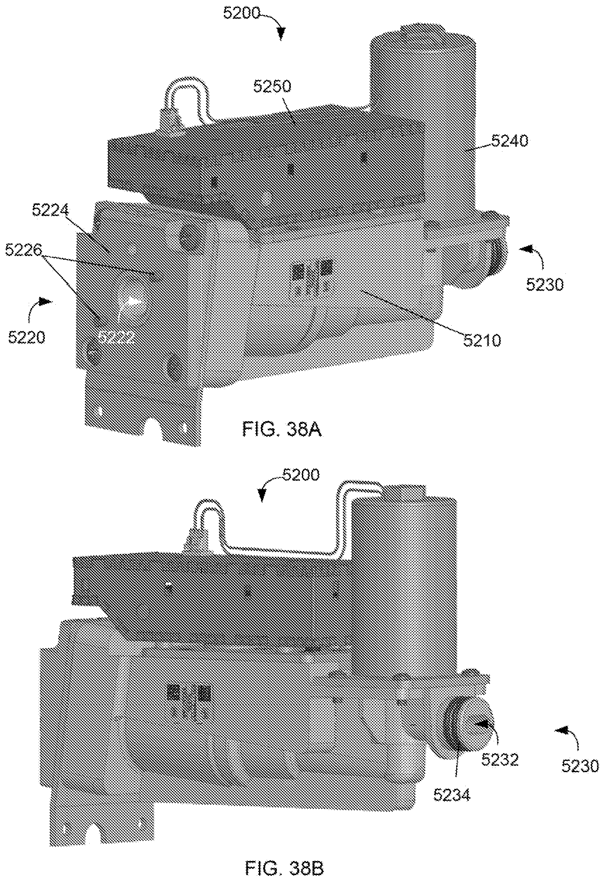

FIGS. 38A-38B depict an exemplary flow cassette 5200 according to certain aspects of the present disclosure. The flow cassette 5200 has a body 5210 with an inlet end 5220 and an outlet end 5230. The inlet end 5220 includes an inlet 5222 that is configured to sealingly mate with a coupler 5122 (not shown in FIG. 38A) of the gas inlet adapter 5120. The inlet end 5220 may also include locating features 5226, for example protruding pins, that align the gas inlet adapter 5120 to the inlet 5222 and a mating face 5224 that provides a reference surface for the mated gas inlet adapter 5120. A solenoid 5240 is attached to the body proximate to the outlet end 5230 and is discussed in greater detail with respect to FIG. 39A. The electronics module 5250 is attached, in this embodiment, to the top of the body 5210. The details of the electronics module are discussed in greater detail with respect to FIG. 37.

FIG. 38B is a reverse-angle view of the flow cassette 5200 that shows the outlet 5232 and the seal 5234, in this example two O-rings, that are arranged at the outlet end 5230. The outlet end 5230 is configured to sealingly mate with other gas passages (not shown in FIG. 38B) within the ventilator 1100.

FIG. 39A is a cross-section of the flow cassette 5200 of FIGS. 38A-38B according to certain aspects of the present disclosure. An enlarged view of the region indicated by the dashed-line box labeled "A" is shown in FIG. 39B.

The dashed-line box 5400 indicates elements of the flow sensor 5400, including the pressure sensors 5420A, 5420B and a flow restriction 5410 that, in this example, is a porous disk. The porous disk 5410 provides a known flow resistance that creates a pressure drop across the porous disk 5410 that varies with flow rate and may be calibrated for one or more gases or gas mixtures. An actual pressure drop can be determined by measuring the pressures upstream and downstream of the porous disk 5410 with the pressure sensors 5420A and 5420B and determining the pressure difference between the pressure measurements. In conjunction with the knowledge of which gas is flowing through the porous disk 5410, derived from the configuration of the gas inlet adapter 5120 as indicated by the magnet 5128 and sensed by the Hall Effect sensor 5258, and the knowledge of the temperature of the gas, as measured by the temperature sensor 5270, the pressure drop can be used to determine the true flow rate, sometimes referred to as "the compensated flow rate," of the gas that is passing through the porous disk 5410. The flow sensor 5400 may also include pressure sensing electronics 5422 that filter and condition the signals from the pressure sensors 5420A, 5420B and may convert the signals to digital form.

The dashed-line box 5300 indicates elements of the proportional valve 5300, including the solenoid 5240 and a plug 5320 that fits into a bore 5310 of the passage 5223. In certain embodiments, the plug 5320 and bore 5310 form an on-off fluid valve and the solenoid 5240 is configured to either fully retract or fully extend the plug 5320 so as to open or close the valve 5300. In certain embodiments, the plug 5320 and bore 5310 form a variable-flow orifice and the solenoid 5240 is configured to adjustably position the plug 5320 with respect to the bore 5310 through a feedback control loop operative within the flow cassette processor 5252 that is operatively coupled to the solenoid 5240. In certain embodiments, the flow cassette processor 5252 may actuate the solenoid 5240 so as to provide a determined flow rate, as sensed by the flow sensor 5400, or a determined pressure at the outlet 5232, as sensed by pressure sensor 5420B.

FIG. 39B is an enlarged view of a portion of FIG. 39A according to certain aspects of the present disclosure. Gas entering the inlet 5222 passes to a check valve 5260 that is configured to allow flow through the passage 5223 toward the outlet 5232 while resisting flow in the opposite direction. The check valve 5260 includes, in this embodiment, a rigid structure 5261 having a plurality of through holes 5263 with a flexible disk 5262 attached to the rigid structure 5261 at the center. Gas flowing from the inlet towards the outlet 5232 (not visible in FIG. 39B) creates a pressure on the upstream side of the flexible disk 5262 that pushes the flexible disk 5262 away from rigid structure 5261, thereby uncovering the through holes 5263 and allowing the gas to flow through the check valve 5260. When the pressures on both sides of the check valve 5260 equalize, the flexible disk 5262 returns to sealing contact with the rigid structure 5261, thereby covering the through holes 5263 and preventing gas from flowing through the check valve towards the inlet 5222.

After passing through the check valve 5260, gas passes through a filter 5264 that, in this embodiment, is formed as a hollow cylinder that is held in place by a cap 5266 having legs 5267 that contact the rigid structure 5261 of the check valve 5260 so as to retain the cap 5266 and filter 5264 in place. Gas passes around the outside of the cylinder and then passes inward through the filter 5264 to the hollow center and then flows out of the filter 5264. In certain embodiments, the filter 5264 comprises a mechanical filter configured to trap particulates above a determined size. In certain embodiments, the filter 5264 comprises one or more chemical filters, for example an activated charcoal or a desiccant, that are configured to absorb certain materials such as water or odors. In this embodiment, the temperature sensing element 5271 is disposed proximate to the filter 5264 and flush with the wall of the passage 5223.

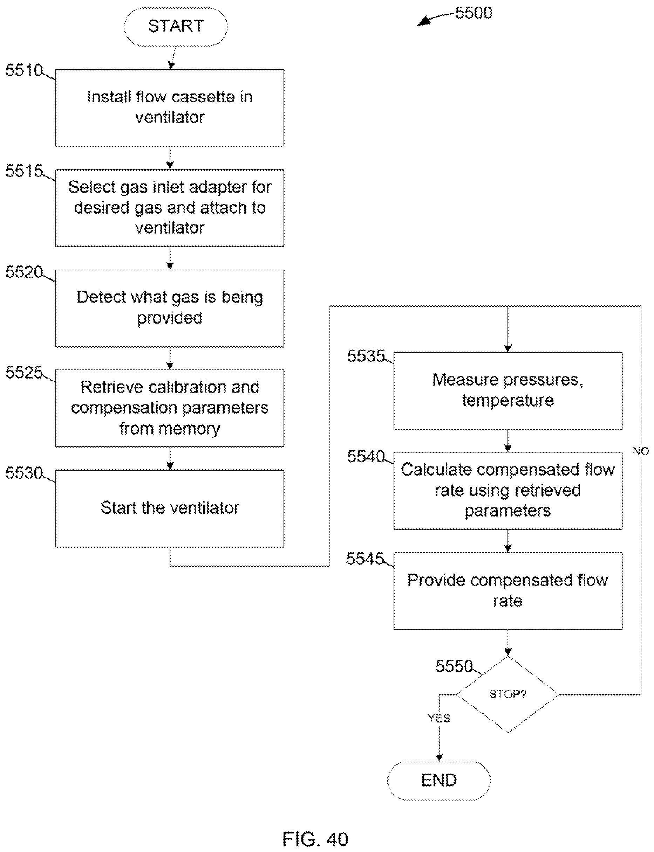

FIG. 40 is a flow chart of an exemplary configuration process 5500 according to certain aspects of the present disclosure. The process 5500 starts in step 5510 by installing a flow cassette 5200 in a ventilator 1100. In step 5515, the user determines which gas or gas mixture, for example oxygen or heliox 70/30, will be provided to the patient, selects the proper gas inlet adapter 5120, and attaches the gas inlet adapter 5120 to the ventilator 1100 in the proper configuration such that the correct connector of the gas inlet adapter 5120 for the determined gas or gas mixture is coupled to the flow cassette 5200. As the gas inlet adapter 5120 includes a magnet 5124 that indicates the type of gas being provided by the specific gas inlet adapter 5120 as installed in the ventilator 1100, the processor 5252 of the flow cassette 5200 can automatically determine in step 5520 which gas or gas mixture is being provided by sensing the magnet 5124 through the Hall Effect sensor 5258, as discussed with respect to FIG. 37. In step 5525, the processor 5252 loads information from the memory 5254 that may include calibration and/or compensation parameters related to the flow sensor 5400. The ventilator 1100 is now configured for use. After the user connects a breathing circuit with a patient, generally as shown in FIG. 10, the user starts the ventilator 1100 in step 5530.

During operation of the ventilator 1100, the flow cassette 5200 measures the pressures on both sides of the porous disk 5410 and the temperature of the gas passing through the flow cassette 5200 in step 5535 using the flow sensor 5400 and temperature sensor 5270, respectively, as described with respect to FIGS. 37 and 39A. In step 5540, the processor 5252 applies the compensation and calibration information downloaded in step 5525 to calculate the actual flow rate of the gas and provides this flow rate information in step 5545, for example to a processor in the ventilator 1100. Step 5550 is a decision point that branches depending on whether a "stop" command has been received. If the user has provided a "stop" command, the process 5500 branches along the "yes" path to the end and terminates. IF the user has not provided a "stop" command, the process 5500 branches along the "no" path back to step 5535 and measures the pressures and temperature. The process 5500 will loop through the steps 5535-5550 until a "stop" command is received.

In summary, it can be seen that the disclosed embodiments of the flow cassette consolidate certain mechanical functions, such as backflow prevention and filtration, and the sensing of certain parameters, such as flow rate, in a compact and modular form. In certain embodiments, the flow cassette includes electronics that process the raw measurements using internally stored compensation and calibration data and provide more accurate values of the sensed parameters. In certain embodiments, the flow cassette may be configured to provide either a determined pressure or a determined flow rate of the supply gas at the outlet. The modular form enables this subsystem to be independently tested and calibrated as well as simplifying assembly and replacement.

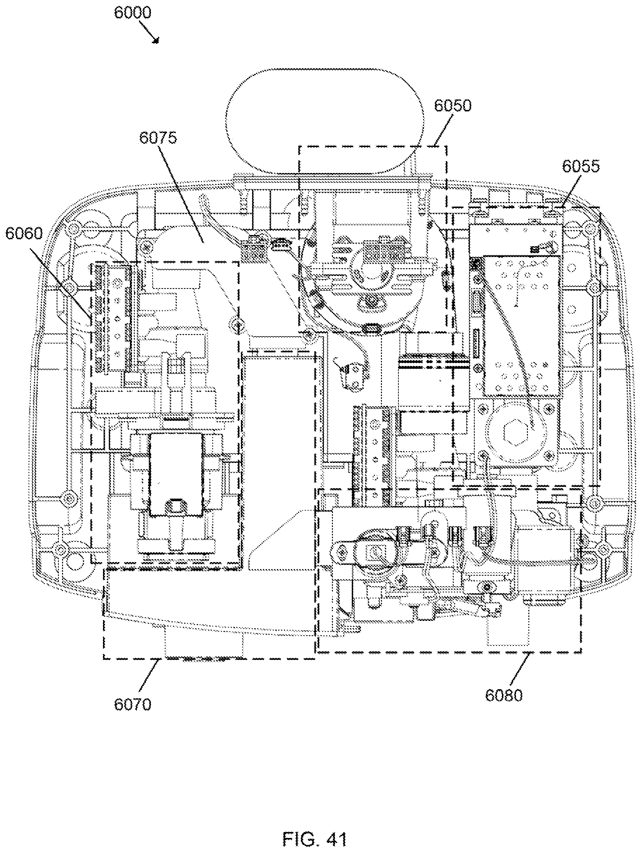

FIG. 41 illustrates a ventilator 6000, which may correspond to the ventilator 1100. The ventilator 600 comprises various component parts, which may each be removed with a common tool, such as a Philips screwdriver. The ventilator 600 comprises a blower 6050, a first flow valve 6055, a second flow valve 6060, a filter chamber 6070, and insulated flow path 6075, and a flow combiner 6080. The component parts may be designed to fit within specific interior dimensions of the ventilator 6000. Each of the component parts may be removed and replaced with alternative component parts, which may have similar dimensions as the part being replaced.

The blower 6050, which may correspond to the blower 350 and/or the blower 750, may pull in gas. The blower 6050 may include a HEPA filter. The first flow valve 6055, which may correspond to the O₂ flow cassette 355 and/or the inflow valve 755, receives another gas, such as pressurized oxygen from a gas supply. The flow combiner 6080 may combine the gases from the blower 6050 and the first flow valve 6055. The flow combiner 6080 may connect to the patient for an inspiration phase. The flow combiner 6080 may include a flow sensor for detecting flow to the patient, a safety valve which may open to protect the patient during an overpressure condition, and an occluder valve which blocks inflow to allow a clinician to measure inspiratory effort by the patient.

For the patient's expiration phase, the patient's expiration flow may be connected to the filter chamber 6070. The filter chamber 6070 may include a chamber, and may also be configured to accumulate condensation from expiration. The filter chamber 6070 may include or be connected to a heater for warming the expiration and reducing condensation. The expiration flow may continue through the insulated flow path 6075, which includes insulation to prevent the expiration from cooling down and causing condensation. The expiration flow continues to the second flow valve 6060, which may correspond to the exhalation valve 360 and/or the exhalation valve 760.

This description is provided to enable any person skilled in the art to practice the various aspects described herein. While the foregoing has described what are considered to be the best mode and/or other examples, it is understood that various modifications to these aspects will be readily apparent to those skilled in the art, and the generic principles defined herein may be applied to other aspects. Thus, the claims are not intended to be limited to the aspects shown herein, but is to be accorded the full scope consistent with the language claims, wherein reference to an element in the singular is not intended to mean "one and only one" unless specifically so stated, but rather "one or more." Unless specifically stated otherwise, the terms "a set" and "some" refer to one or more. Pronouns in the masculine (e.g., his) include the feminine and neuter gender (e.g., her and its) and vice versa. Headings and subheadings, if any, are used for convenience only and do not limit the invention.

It is understood that the specific order or hierarchy of steps in the processes disclosed is an illustration of exemplary approaches. Based upon design preferences, it is understood that the specific order or hierarchy of steps in the processes may be rearranged. Some of the steps may be performed simultaneously.

Terms such as "top," "bottom," "front," "rear" and the like as used in this disclosure should be understood as referring to an arbitrary frame of reference, rather than to the ordinary gravitational frame of reference. Thus, a top surface, a bottom surface, a front surface, and a rear surface may extend upwardly, downwardly, diagonally, or horizontally in a gravitational frame of reference.

A phrase such as an "aspect" does not imply that such aspect is essential to the subject technology or that such aspect applies to all configurations of the subject technology. A disclosure relating to an aspect may apply to all configurations, or one or more configurations. A phrase such as an aspect may refer to one or more aspects and vice versa. A phrase such as an "embodiment" does not imply that such embodiment is essential to the subject technology or that such embodiment applies to all configurations of the subject technology. A disclosure relating to an embodiment may apply to all embodiments, or one or more embodiments. A phrase such an embodiment may refer to one or more embodiments and vice versa.

The word "exemplary" is used herein to mean "serving as an example or illustration." Any aspect or design described herein as "exemplary" is not necessarily to be construed as preferred or advantageous over other aspects or designs.

All structural and functional equivalents to the elements of the various aspects described throughout this disclosure that are known or later come to be known to those of ordinary skill in the art are expressly incorporated herein by reference and are intended to be encompassed by the claims. Moreover, nothing disclosed herein is intended to be dedicated to the public regardless of whether such disclosure is explicitly recited in the claims. No claim element is to be construed under the provisions of 35 U.S.C. §112, sixth paragraph, unless the element is expressly recited using the phrase "means for" or, in the case of a method claim, the element is recited using the phrase "step for." Furthermore, to the extent that the term "include," "have," or the like is used in the description or the claims, such term is intended to be inclusive in a manner similar to the term "comprise" as "comprise" is interpreted when employed as a transitional word in a claim.

This specification describes example aspects of the subject technology, which may include at least the following concepts:
Concept 1. A ventilator comprising: a blower comprising: a housing defining an impeller cavity; an impeller plate disposed within the impeller cavity and comprising an outside edge; and one or more vanes disposed on the impeller plate and comprising a leading surface and a trailing surface connecting at a tip; and a flow control device comprising: a fixed magnetic field; a drive coil configured to move within the fixed magnetic field in response to a low frequency signal and configured to receive a high frequency signal; a detection coil adjacent the drive coil and configured to detect the high frequency signal in the drive coil, the detected high frequency signal corresponding to a position of the drive coil; and a processor coupled to the high frequency source and the low frequency source and configured (i) to receive the detected high frequency signal form the detection coil and (ii) to adjust the low frequency signal to move the drive coil, wherein the blower is in fluid communication with the flow control device.
Concept 1 may or may not provide that the leading surface comprises a first portion abutting a second portion, the first portion extends from the tip with a first radius, the second portion extends from the tip with a second radius, the first radius is smaller than the second radius.
Concept 2. The ventilator of Concept 1, wherein the blower is before the flow control device in a fluid path of the ventilator.
Concept 3. The ventilator of Concept 1, wherein the blower is after the flow control device in a fluid path of the ventilator.
Concept 4. The ventilator of Concept 1, further comprising a sensor configured to provide sensor information of the blower and the flow control device, and a ventilation control system configured to send the sensor information to the flow control device.
Concept 5. The ventilator of Concept 1, further comprising a flow cassette in fluid communication with the blower.
Concept 6. The ventilator of Concept 1, further comprising a fluid inlet adapter configured to removably connect to the flow control device.
Concept 7. The ventilator of Concept 1, wherein the one or more vanes comprises a top surface between the leading and trailing surfaces, the one or more vanes comprises a centerline bisecting the top surface between, and a width measured perpendicular to the centerline varies along the centerline.
Concept 8. The ventilator of Concept 7, wherein each of the one or more vanes have the same width along respective centerlines such that at a distance from the respective tip along the respective centerline, the width of each vane is the same.
Concept 9. A ventilator comprising: a flow cassette comprising: a fluid passage terminating at an inlet and an outlet; a temperature sensor disposed within the fluid passage and configured to detect a temperature; a flow rate sensor disposed within the fluid passage and configured to detect a flow rate; and a processor configured to determine a compensated flow rate based on the temperature and the flow rate; and a fluid inlet adapter comprising: a first inlet end configured to connect to a fluid source; a second inlet end configured to removably connect to the inlet of the flow cassette; a latching component configured to secure the fluid inlet adapter to the flow cassette; and a machine-readable indicator for identifying the fluid source.
Concept 9 may or may not provide that the temperature sensor is disposed within the fluid passage, that the flow rate sense is disposed within the fluid passage, or that the cassette includes a processor configured to determine a compensated flow rate based on the temperature and the flow rate.
Concept 10. The ventilator of Concept 9, wherein the machine-readable indicator comprises one or more magnets.
Concept 11. The ventilator of Concept 10, wherein the ventilator comprises a sensor configured to detect a configuration of the one or more magnets.
Concept 12. The ventilator of Concept 9, wherein the second inlet end of the fluid inlet adapter comprises a threaded nut.
Concept 13. The ventilator of Concept 12, wherein the inlet of the flow cassette comprises a threaded connector configured to interface with the threaded nut.
Concept 14. The ventilator of Concept 9, wherein the flow cassette comprises a valve disposed within the fluid passage and configured to provide a selectable flow restriction.
Concept 15. The ventilator of Concept 9, further comprising a flow control device comprising: a drive coil configured to move in response to a low frequency signal; a detection coil configured to detect a position of the drive coil based on a high frequency signal; a flow control device processor configured to receive the high frequency signal and modify the low frequency signal; and a valve orifice defining a variable opening, the variable opening adjustable based on the position of the drive coil.
Concept 16. The ventilator of Concept 15, wherein the fluid inlet adapter comprises a threaded nut configured to interface with the threaded connector of the flow control device.
Concept 17. A ventilator comprising: a flow sensor comprising: a fluid passage; a flow restriction disposed within the fluid passage such that a fluid passing through the fluid passage must pass through the flow restriction; a first pressure sensor coupled to a first end of the fluid passage and configured to detect a first pressure; a second pressure sensor coupled to a second end of the fluid passage and configured to detect a second pressure, such that the flow restriction is between the first and second pressure sensors; a temperature sensor coupled to the fluid passage and configured to detect a temperature; and a flow sensor processor configured to determine a compensated flow rate based at least on the first pressure, the second pressure, and the temperature.
Concept 18. The ventilator of Concept 17, further comprising a fluid inlet adapter comprising a machine-readable indicator for identifying a fluid source and an inlet in flow communication with the flow sensor.
Concept 19. The ventilator of Concept 18, wherein the flow sensor processor is further configured to receive an identification of the fluid based on the machine-readable indicator and determine the compensated flow rate based at least on the identified fluid.
Concept 20. The ventilator of Concept 17, further comprising a blower in fluid communication with the flow sensor, the blower comprising: an impeller plate; and one or more vanes disposed on the impeller plate and comprising a leading surface and a trailing surface connecting at a tip, wherein the leading surface comprises a first portion abutting a second portion, the first portion extends from the tip with a first radius, the second portion extends from the tip with a second radius, the first radius is smaller than the second radius.

## Claims

1. A ventilator (1100) comprising:
a blower (750, 100) comprising:
a housing (112) defining an impeller cavity (119);
an impeller plate (162) disposed within the impeller cavity and comprising an outside edge (164); and
one or more vanes (170, 171) disposed on the impeller plate and comprising a leading surface (180) and a trailing surface (182) connecting at a tip (174); and
a flow control device (500) **characterized in that** it comprises:
a fixed magnetic field (525);
a drive coil (520, 615) configured to move within the fixed magnetic field in response to a low frequency signal and configured to receive a high frequency signal;
a detection coil (540) adjacent the drive coil and configured to detect the high frequency signal in the drive coil, the detected high frequency signal corresponding to a position of the drive coil; and
a processor (545) coupled to the high frequency source (505) and the low frequency source (510) and configured (i) to receive the detected high frequency signal form the detection coil and (ii) to adjust the low frequency signal to move the drive coil, wherein the blower is in fluid communication with the flow control device.

2. The ventilator (1100) of Claim 1, wherein the blower (750, 100) is before the flow control device (500) in a fluid path of the ventilator (1100).

3. The ventilator (1100) of Claim 1, wherein the blower (750, 100) is after the flow control device in a fluid path of the ventilator (1100).

4. The ventilator (1100) of Claim 1, further comprising a sensor (365) configured to provide sensor information of the blower (750, 100) and the flow control device (500), and a ventilation control system (330) configured to send the sensor information to the flow control device (500).

5. The ventilator (1100) of Claim 1, further comprising a flow cassette (355) in fluid communication with the blower (750, 100).

6. The ventilator (1100) of Claim 1, further comprising a fluid inlet adapter (2100) configured to removably connect to the flow control device (500).

7. The ventilator (1100) of Claim 1, wherein the one or more vanes (170, 171) comprises a top surface between the leading and trailing surfaces, the one or more vanes (170, 171) comprises a centerline (179) bisecting the top surface between, and a width measured perpendicular to the centerline varies along the centerline.

8. The ventilator (1100) of Claim 7, wherein each of the one or more vanes (170, 171) have the same width along respective centerlines such that at a distance from the respective tip along the respective centerline, the width of each vane (170, 171) is the same.

## Patentansprüche

1. Beatmungsgerät (1100), umfassend:
ein Gebläse (750, 100) mit:
einem Gehäuse (112), das eine Laufradkammer (119) bildet;
einer Laufradscheibe (162), die in der Laufradkammer angeordnet ist und einen Außenrand (164) aufweist; und
einer oder mehreren Schaufeln (170, 171), die an der Laufradscheibe angeordnet ist bzw. sind und eine Vorderfläche (180) sowie eine sich an eine Spitze (174) anschließende Rückfläche (182) aufweist bzw. aufweisen; und
eine Durchflusssteuereinheit (500), **dadurch gekennzeichnet, dass** sie aufweist;
ein ortsfestes Magnetfeld (525);
eine Antriebsspule (520, 615), die dazu ausgelegt ist, sich im Ansprechen auf ein Niederfrequenzsignal innerhalb des ortsfesten Magnetfelds zu bewegen, und dazu ausgelegt ist, ein Hochfrequenzsignal zu empfangen;
eine Erfassungsspule (540), die an die Antriebsspule angrenzt und dazu ausgelegt ist, das Hochfrequenzsignal in der Antriebsspule zu erfassen, wobei das erfasste Hochfrequenzsignal einer Position der Antriebsspule entspricht; und
einen Prozessor (545), der an die Hochfrequenzquelle (505) und die Niederfrequenzquelle (510) angeschlossen und dazu ausgelegt ist, (i) das von der Erfassungsspule erfasste Hochfrequenzsignal zu empfangen und (ii) das Niederfrequenzsignal zur Bewegung der Antriebsspule einzustellen, wobei das Gebläse mit der Durchflusssteuereinheit in Fluidverbindung steht.

2. Beatmungsgerät (1100) nach Anspruch 1, wobei das Gebläse (750, 100) in einem Fluidweg des Beatmungsgeräts (1100) vor der Durchflusssteuereinheit (500) liegt.

3. Beatmungsgerät (1100) nach Anspruch 1, wobei das Gebläse (750, 100) in einem Fluidweg des Beatmungsgeräts (1100) hinter der Durchflusssteuereinheit (500) liegt.

4. Beatmungsgerät (1100) nach Anspruch 1, darüber hinaus einen Sensor (365) umfassend, der dazu ausgelegt ist, Sensorinformationen des Gebläses (750, 100) und der Durchflusssteuereinheit (500) bereitzustellen, und ein Beatmungsgerät-Steuersystem (330) aufweisend, das dazu ausgelegt ist, die Sensorinformationen an die Durchflusssteuereinheit (500) zu senden.

5. Beatmungsgerät (1100) nach Anspruch 1, darüber hinaus eine Durchflusskassette (355) aufweisend, die mit dem Gebläse (750, 100) in Fluidverbindung steht.

6. Beatmungsgerät (1100) nach Anspruch 1, darüber hinaus einen Fluideinlassadapter (2100) umfassend, der dazu ausgelegt ist, abnehmbar an die Durchflusssteuereinheit (500) angeschlossen zu sein.

7. Beatmungsgerät (1100) nach Anspruch 1, wobei die eine oder die mehreren Schaufeln (170, 171) eine Oberseite zwischen der Vorderfläche und Rückfläche aufweist bzw. aufweisen, die eine oder die mehreren Schaufeln (170, 171) eine Mittellinie (179) aufweist bzw. aufweisen, durch welche die Oberseite zweigeteilt wird, und eine senkrecht zur Mittellinie gemessene Breite sich entlang der Mittellinie ändert.

8. Beatmungsgerät (1100) nach Anspruch 7, wobei die eine oder die mehreren Schaufeln (170, 171) entlang jeweiligen Mittellinien dieselbe Breite hat bzw. haben, derart, dass in einem Abstand von einer jeweiligen Spitze entlang der jeweiligen Mittellinie die Breite jeder Schaufel (170, 171) dieselbe ist.

## Revendications

1. Ventilateur (1100), comprenant :
une soufflante (750, 100) comprenant :
un logement (112) définissant une cavité d'impulseur (119) ;
une plaque d'impulseur (162) disposée dans la cavité d'impulseur et comprenant un bord extérieur (164), et
une ou plusieurs ailettes (170, 171) disposée sur la plaque d'impulseur et comprenant une surface avant (180) et une surface arrière (182) se rejoignant au niveau d'une pointe (174), et un dispositif de régulation d'écoulement (500), **caractérisé en ce qu'**il comprend :
un champ magnétique fixé (525) ;
une bobine d'excitation (520, 615) configurée pour se déplacer dans le champ magnétique fixé en réaction à un signal basse fréquence et configurée pour recevoir un signal haute fréquence ;
une bobine de détection (540), adjacente à la bobine d'excitation, et configurée pour détecter le signal haute fréquence dans la bobine d'excitation, le signal haute fréquence détecté correspondant à une position de la bobine d'excitation ; et
un processeur (545) couplé à la source haute fréquence (505) et la source basse fréquence (510) et configuré (i) pour recevoir le signal haute fréquence détecté de la bobine de détection, et (ii) pour ajuster le signal basse fréquence pour déplacer la bobine d'excitation, dans lequel la soufflante présente une communication de fluide avec le dispositif de régulation d'écoulement.

2. Ventilateur (1100) selon la revendication 1, dans lequel la soufflante (750, 100) se trouve avant le dispositif de régulation d'écoulement (500) dans un trajet de fluide du ventilateur (1100).

3. Ventilateur (1100) selon la revendication 1, dans lequel la soufflante (750, 100) se trouve après le dispositif de régulation d'écoulement dans un trajet de fluide du ventilateur (1100).

4. Ventilateur (1100) selon la revendication 1, comprenant en outre un capteur (365) configuré pour fournir des informations de capteur de la soufflante (750, 100) et du dispositif de régulation d'écoulement (500), et un système de régulation de ventilation (330) configuré pour envoyer des informations de capteur au dispositif de régulation d'écoulement (500).

5. Ventilateur (1100) selon la revendication 1, comprenant en outre une cassette d'écoulement (355) présentant une communication de fluide avec la soufflante (750, 100).

6. Ventilateur (1100) selon la revendication 1, comprenant en outre un adaptateur d'admission de fluide (2100) configuré pour être raccordé de manière amovible au dispositif de régulation d'écoulement (500).

7. Ventilateur (1100) selon la revendication 1, dans lequel les une ou plusieurs ailettes (170, 171) comprennent une surface supérieure entre les surfaces avant et arrière, les une ou plusieurs ailettes (170, 171) comprenant une ligne médiane (179) bissectant la surface supérieure au milieu, et une largeur mesurée perpendiculairement à la ligne médiane varie le long de la ligne médiane.

8. Ventilateur (1100) selon la revendication 7, dans lequel chacune des une ou plusieurs ailettes (170, 171) présentent la même largeur le long de lignes médianes respectives de sorte qu'à une distance à partir de la pointe respective le long de la ligne médiane respective, une largeur de chaque ailette (170, 171) est identique.
